(19) Europäisches Patentamt
European Patent Office
Office européen des brevets

(11) EP 1 662 007 B1

(12) **EUROPEAN PATENT SPECIFICATION**

(45) Date of publication and mention of the grant of the patent:
**18.05.2011 Bulletin 2011/20**

(21) Application number: **04772572.6**

(22) Date of filing: **25.08.2004**

(51) Int Cl.:
***A61K 39/395*** *(2006.01)* ***A61P 1/04*** *(2006.01)*

(86) International application number:
**PCT/JP2004/012616**

(87) International publication number:
**WO 2005/021792 (10.03.2005 Gazette 2005/10)**

(54) **PREVENTIVE OR REMEDY FOR INFLAMMATORY BOWEL DISEASES CONTAINING ANTI-CD81 ANTIBODY AS THE ACTIVE INGREDIENT**

MITTEL ZUR VORBEUGUNG ODER HEILUNG ENTZÜNDLICHER DARMERKRANKUNGEN MIT ANTI-CD81-ANTIKÖRPER ALS AKTIVEM INHALTSSTOFF

PRODUIT PROPHYLACTIQUE OU REMEDE CONTRE LA MALADIE INTESTINALE INFLAMMATOIRE, CONTENANT UN ANTICORPS ANTI-CD81 COMME PRINCIPE ACTIF

(84) Designated Contracting States:
**AT BE BG CH CY CZ DE DK EE ES FI FR GB GR HU IE IT LI LU MC NL PL PT RO SE SI SK TR**

(30) Priority: **28.08.2003 JP 2003304264**

(43) Date of publication of application:
**31.05.2006 Bulletin 2006/22**

(73) Proprietor: **Dainippon Sumitomo Pharma Co., Ltd.**
**Osaka 541-8524 (JP)**

(72) Inventors:
- **WATANABE, Takamasa**
  **Osaka-shi,**
  **Osaka 5540022 (JP)**
- **KIKUCHI, Kaoru**
  **Osaka-shi,**
  **Osaka 5540022 (JP)**

(74) Representative: **Vossius & Partner**
**Siebertstrasse 4**
**81675 München (DE)**

(56) References cited:
**WO-A1-00/67796**
**WO-A1-01/58459**
**WO-A1-98/25647**
**WO-A1-99/18198**
**WO-A2-02/22212**
**WO-A2-03/053342**
**JP-A- 2001 299 140**
**JP-A- 2003 005 093**
**JP-A- 2003 230 388**
**JP-A- 2003 235 573**

- **MAECKER H T ET AL: "DIFFERENTIAL EXPRESSION OF MURINE CD81 HIGHLIGHTED BY NEW ANTI-MOUSE CD81 MONOCLONAL ANTIBODIES" HYBRIDOMA, LIEBERT, NEW YORK, NY, US, vol. 19, no. 1, February 2000 (2000-02), pages 15-22, XP009005201 ISSN: 0272-457X**
- **KRONENBERGER B. ET AL.: 'Interferon alfa down-regulates CD81 in patients with chronic hepatitis C' HEPATOOLOGY vol. 33, no. 6, 2001, pages 1518 - 1526, XP008031935**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Sumitomo Pharmaceuticals Co., Ltd.

<120> Method of preventing or treating inflammatory bowel diseases containing as the ingredient CD81 antibodies

<130> 533734

<150> JP 2003-304264
<151> 2003-08-28

<160> 2

<170> PatentIn version 3.2

<210> 1
<211> 1332
<212> DNA
<213> Homo sapiens

<400> 1

ccggcccgcg ccccgcaggc cgcccgccgc ccgcgccgcc atgggagtgg agggctgcac 60

caagtgcatc aagtacctgc tcttcgtctt caatttcgtc ttctggctgg ctggaggcgt 120

gatcctgggt gtggccctgt ggctccgcca tgacccgcag accaccaacc tcctgtatct 180

ggagctggga gacaagcccg cgcccaacac cttctatgta ggcatctaca tcctcatcgc 240

tgtgggcgct gtcatgatgt tcgttggctt cctgggctgc tacggggcca tccaggaatc 300

ccagtgcctg ctggggacgt tcttcacctg cctggtcatc ctgtttgcct gtgaggtggc 360

cgccggcatc tggggctttg tcaacaagga ccagatcgcc aaggatgtga agcagttcta 420

tgaccaggcc ctacagcagg ccgtggtgga tgatgacgcc aacaacgcca aggctgtggt 480

gaagaccttc cacgagacgc ttgactgctg tggctccagc acactgactg ctttgaccac 540

ctcagtgctc aagaacaatt tgtgtccctc gggcagcaac atcatcagca acctcttcaa 600

ggaggactgc caccagaaga tcgatgacct cttctccggg aagctgtacc tcatcggcat 660

tgctgccatc gtggtcgctg tgatcatgat cttcgagatg atcctgagca tggtgctgtg 720

ctgtggcatc cggaacagct ccgtgtactg aggccccgca gctctggcca cagggacctc 780

tgcagtgccc cctaagtgac ccggacactt ccgagggggc catcaccgcc tgtgtatata 840

acgtttccgg tattactctg ctacacgtag ccttttttact tttggggttt tgtttttgtt 900

ctgaactttc ctgttacctt ttcagggctg acgtcacatg taggtggcgt gtatgagtgg 960

agacgggcct gggtcttggg gactggaggg caggggtcct tctgccctgg ggtcccaggg 1020

tgctctgcct gctcagccag gcctctcctg ggagccactc gcccagagac tcagcttggc 1080

caacttgggg ggctgtgtcc acccagcccg cccgtcctgt gggctgcaca gctcaccttg 1140

ttccctcctg ccccggttcg agagccgagt ctgtgggcac tctctgcctt catgcacctg 1200

tcctttctaa cacgtcgcct tcaactgtaa tcacaacatc ctgactccgt catttaataa 1260

agaaggaaca tcaggcatgc taaaaaaaaa aaaaaaaaaa aaaaaaaaaa aaaaaaaaaa 1320

aaaaaaaaaa aa 1332

<210> 2
<211> 236
<212> PRT
<213> Homo sapiens

<400> 2

Met Gly Val Glu Gly Cys Thr Lys Cys Ile Lys Tyr Leu Leu Phe Val
1 5 10 15

Phe Asn Phe Val Phe Trp Leu Ala Gly Gly Val Ile Leu Gly Val Ala
20 25 30

Leu Trp Leu Arg His Asp Pro Gln Thr Thr Asn Leu Leu Tyr Leu Glu
35 40 45

Leu Gly Asp Lys Pro Ala Pro Asn Thr Phe Tyr Val Gly Ile Tyr Ile
50 55 60

Leu Ile Ala Val Gly Ala Val Met Met Phe Val Gly Phe Leu Gly Cys
65 70 75 80

Tyr Gly Ala Ile Gln Glu Ser Gln Cys Leu Leu Gly Thr Phe Phe Thr
85 90 95

Cys Leu Val Ile Leu Phe Ala Cys Glu Val Ala Ala Gly Ile Trp Gly
100 105 110

Phe Val Asn Lys Asp Gln Ile Ala Lys Asp Val Lys Gln Phe Tyr Asp
115 120 125

Gln Ala Leu Gln Gln Ala Val Val Asp Asp Asp Ala Asn Asn Ala Lys
130 135 140

Ala Val Val Lys Thr Phe His Glu Thr Leu Asp Cys Cys Gly Ser Ser
145 150 155 160

Thr Leu Thr Ala Leu Thr Thr Ser Val Leu Lys Asn Asn Leu Cys Pro
165 170 175

Ser Gly Ser Asn Ile Ile Ser Asn Leu Phe Lys Glu Asp Cys His Gln
180 185 190

Lys Ile Asp Asp Leu Phe Ser Gly Lys Leu Tyr Leu Ile Gly Ile Ala
195 200 205

Ala Ile Val Val Ala Val Ile Met Ile Phe Glu Met Ile Leu Ser Met

210 215 220

Val Leu Cys Cys Gly Ile Arg Asn Ser Ser Val Tyr
225 230 235

## Description

TECHNICAL FIELD

Herein disclosed is a method for screening a substance effective for preventing, improving or treating inflammatory bowel diseases (hereinafter sometimes referred to as "IBD"), the substance obtained by the method, and an agent for preventing, improving or treating IBD, containing the substance obtained by the method or an anti-CD81 antibody as an active ingredient.

Further disclosed is a disease marker useful for diagnosis of inflammatory bowel diseases (IBD), and a detecting method (diagnosing method) of IBD using the disease marker.

Background Art

Bowels are organs which digest and absorb nutrients and water essential for activities of life of organisms. Meanwhile, they are also organs which have an immunodefense performance for excluding foreign matters such as pathogens and keep life conservation by controlling contradictory qualities in a well-balanced manner. It is however known that when the balance of these functions becomes abnormal, this dynamic equilibrium is broken to induce various bowel diseases. Especially, regarding inflammatory bowel diseases (abbreviated as IBD) of which patients have been increased in number in recent years, a pharmacotherapy using 5-aminosalicylic acid pharmaceutical preparations such as sulfasalazine, steroids and the like cannot provide satisfactory therapeutic effects. Accordingly, the diseases have been treated by enterectomy, leukapheresis or the like, and more effective agents have been thus required.

Inflammatory bowel diseases (IBD) are, in view of pathogenic states thereof, grouped into ulcerative colitis (abbreviated as UC) and Crohn's disease (abbreviated as CD). In recent years, it has been known that protein pharmaceutical preparations such as an anti-TNF antibody are effective as a therapeutic agent of Crohn's disease (Lancet. 342, 173-174, 1993). Nevertheless, they are costly agents, and are applied to only patients with a steroid resistance. An effect against ulcerative colitis is also weak. Thus, a satisfactory medical treatment has not yet been presented. The inflammatory bowel diseases are diseases which repeat remission and recrudescence, are very poor in QOL (=Quality Of Life), and are still low in degree of satisfaction regarding pharmacotherapy. Accordingly, the development of an effective therapeutic agent has been in high demand in a medical care.

Further, a reaction to pharmacotherapy varies with individual patients, and there are inflammatory bowel disease (IBD) patients unreactive to pharmacotherapy in all therapeutic agents which have been currently used. In view of the analysis of gene polymorphism and disease sensitivity, there are cases in which polymorphism of interleukin-1 or polymorphism of NOD2 gene is considered to be a disease causal gene. In basic medical studies also, it is being clarified that there is a difference in genetic background for attack of the pathogenic state among individual patients (Current Opinion in Anti-inflammatory & Immunomodulatory Investigational Drugs. 2, 272-274, 2000).

In the recent medical care, it has been required to exactly select a therapeutic method to adapt to symptoms of individual patients without limiting it to inflammatory bowel diseases (IBD). In recent years, the necessity for improvement of QOL (Quality of life) in society of the advanced age has been recognized, and it has been earnestly demanded to apply not a therapy common to all patients but an appropriate therapy to adapt to symptoms of individual patients. For conducting such a so-called tailor-made therapy, a disease marker that exactly reflects symptoms of individual patients or causes (genetic backgrounds) thereof is profitable, and studies intended for the search and development thereof have been assiduously conducted at present.

CD81 is a surface molecule of 26 kDa which is expressed in wide-ranging cells, and has an activity of decreasing a threshold of B cell activation by forming a complex with CD21, CD19 and Leu 13 in a B cell. A T cell is associated with CD4 and CD8 to transmit a stimulus information into cells. In view of these matters, CD81 is considered to have a significant role in an immune response to a heteroantigen. Moreover, it is involved in various integrins physiologically and functionally to activate VLA-4 ($\alpha 4 \beta 1$ integrin) in a B cell or LFA-1 ($\alpha L \beta 2$ integrin) in a thymocyte.

As a disease associated with CD81, hepatitis C type is mentioned. It has been reported that screening of an E2 binding molecule is conducted from a cDNA expression library derived from a human T lymphoma (cell clone having a high affinity with E2) using an envelope protein E2 region of hepatitis C type virus (HCV) as a probe to bind a human CD81 molecule to E2 and that an E2 binding region of such a CD81 molecule is bound to an RNA of HCV and this binding is inhibited with an antibody of neutralizing HCV infection (Science 282, 938-941, 1998). That is, it has been known that CD81 acts as a receptor of HCV to participate in triggering of hepatitis C type.

It has also been known that an autoimmune disease becomes serious by HCV infection, and it has been reported that CD81 is related with a degree of seriousness in chronic HCV infection and CD81 is related with a rheumatic factor (Clinical & Experimental Immunology. 128 (2) : 353-8, 2000). There is also a report stating that HCV infection may be a dangerous factor of inflammatory bowel diseases (Gastroenterologie Clinique et Biologique. 24(1): 77-81, 2000).

Disclosure of the Invention

Herein disclosed is a method for screening an agent useful for preventing, improving or treating inflammatory bowel diseases. It is an object of the invention to provide an agent for preventing, improving or treating the diseases.
Also disclosed is a disease marker useful for diagnosing or treating inflammatory bowel diseases, and a detecting method (gene diagnostic method) of inflammatory bowel diseases using the disease marker.
The present inventors have assiduously conducted investigations to solve the foregoing problems, and have consequently found that the expression of a CD81 gene whose relation with inflammatory bowel diseases (IBD) was hitherto unknown is significantly increased in IBD-pathogenic cells in comparison to IBD-non-pathogenic cells. As will be described in Examples, IBD-pathogenic cells mean a cell population having an ability to induce IBD by transferred into a normal animal, and IBD-non- pathogenic cells mean a cell population that does not induce IBD even by transferred into a normal animal. Specifically, the IBD- pathogenic cells include cells obtained by incubation of lymphatic cells derived from an IBD-attacked mouse through stimulation with Staphylococcal enterotoxin B (SEB). The IBD-non-pathogenic cells include cells obtained by incubation of lymphatic cells derived from an IBD-attacked mouse without stimulation or through stimulation with SEB in the presence of 3-chloro-2-[1-(4-morpholin-4-ylphenyl)cyclobutyl]-4,5,6,7-tetrahydropyrazolo[ 1,5-a]pyrimidine (hereinafter sometimes abbreviated as "compound A"), a compound of JP-A-2002-161084 whose activity of inhibiting an IBD-inducibility is known.
The present inventors have further found that compound A inhibits formation of interferon $\gamma$, an inflammatory cytokine in CD81-positive cells of CD3-positive T cells, and have clarified that an anti-CD81 antibody is effective for treatment of inflammatory bowel disease model animals.
As stated above, the present inventors have clarified that a screening system using inhibition of expression of a CD81 gene, inhibition of expression of a protein encoded by the gene or inhibition of a function (activity) thereof as an index is effective for searching an agent for preventing, improving or treating inflammatory bowel diseases (IBD) based on a new mechanism. They have further obtained findings that an anti-CD81 antibody is useful as an agent for preventing, improving or treating inflammatory bowel diseases and a CD81 gene or an expression product (protein) of CD81 is an excellent disease marker of inflammatory bowel diseases (IBD).
The invention has been completed on the basis of such findings.
Accordingly, the present invention relates to the use of an anti-CD81 antibody for the preparation of a pharmaceutical composition for preventing, improving or treating inflammatory bowel disease (IBD). The present invention also relates to an anti-CD81 antibody for use in preventing, improving or treating inflammatory bowel disease (IBD). Preferably, the CD81 is a mammal CD81. The anti-CD81 antibody is preferably a monoclonal antibody. The inflammatory bowel disease (IBD) to be prevented, improved or treated may be ulcerative colitis or Crohn' s disease.
Furthermore, herein disclosed is:

(1) a method for screening a substance reducing expression of CD81 gene, comprising the following steps (a), (b) and (c):

(a) a step of contacting a test substance with cells capable of expressing CD81 gene,
(b) a step of measuring an amount of CD81 gene expression in the cells contacted with the test substance, and comparing the amount with an amount of the corresponding gene expression in control cells not contacted with the test substance, and
(c) a step of selecting the test substance reducing the amount of CD81 gene expression on the basis of the comparison results in (b),

(2) a method for screening a substance reducing expression of CD81, comprising the following steps (a), (b) and (c):

(a) a step of contacting a test substance with cells capable of expressing CD81,
(b) a step of measuring an expression amount of CD81 in the cells contacted with the test substance, and comparing the expression amount with an expression amount of the protein in control cells not contacted with the test substance, and
(c) a step of selecting the test substance reducing the expression amount of CD81 on the basis of the comparison results in (b),

(3) a method for screening a substance inhibiting function (activity) of CD81, comprising the following steps (a), (b) and (c):

(a) a step of contacting a test substance with CD81,
(b) a step of measuring function (activity) of CD81 caused by the step in (a) above, and comparing the function (activity) with function (activity) of CD81 not contacted with the test substance, and
(c) a step of selecting the test substance inhibiting the function (activity) of CD81 on the basis of the comparison results in (b),

(4) a method for screening of the above item (3), wherein the function (activity) of CD81 is a fibronectin-binding activity, function
(5) a method for screening of the above item (3), wherein the function (activity) of CD81 is a cell-binding activity, function
(6) a method for screening of any one of the above item (3) to (5), wherein B cell is used as a cell expressing CD81, (5), a method for screening an agent as an active ingredient for preventing, improving or treating IBD described in any one of the above items (1) to (6),
(8) an agent for preventing, improving or treating IBD, comprising a substance which reduces expression of CD81 gene as active ingredient,
(9) the agent for preventing, improving or treating IBD of the above item (8), wherein the substance which reduces expression of CD81 gene is obtainable by the method of the above item (1),
(10) an agent for preventing, improving or treating IBD, comprising a substance which reduces expression of CD81 or function (activity) of CD81 as an active ingredient,
(11) the agent for preventing, improving or treating IBD of the above item (10), wherein the substance which reduces expression of CD81 or function (activity) of CD81 is obtainable by the method of the above item (2) or (3),
(12) the agent for preventing, improving or treating IBD of the above item (10), which comprises an anti-CD81 antibody as the active ingredient,
(13) the agent for preventing, improving or treating IBD of the above item (12), wherein the anti-CDB1 antibody is an antibody against mammal CD81,
(14) the agent for preventing, improving or treating IBD of the above item (8), which comprises antisense polynucleotides which contains a base sequence complementary or substantially complementary to the base sequence of CD81 or a part thereof, as the active ingredient,
(15) a disease marker for inflammatory bowel disease (hereinafter, IBD), which comprises a polynucleotide having at 15 continuous bases and / or a polynucleotide complementary thereto in the base sequence of CD81 gene,
(16) the disease marker of the above item (15), which is used as a probe or a primer for diagnosing IBD,
(17) a method for diagnosing IBD, which comprises the following steps (a), (b) and (c): .

(a) a step of binding a RNA prepared from a biopsy of a subject or a complementary polynucleotide transcribed therefrom to the disease marker of the above item (15) or (16),
(b) a step of measuring the RNA derived from the biopsy or the complementary polynucleotide transcribed from the RNA wherein the RNA or the polynucleotide is bound to the disease marker, by using the disease marker as an index, and
(c) a step of judging the attack of IBD on the basis of the measurement results in (b),

(18) the method for diagnosing IBD of the above item (17), wherein the step of judging the attack of IBD described in (c) is performed by comparing the measurement result of the subject with a measurement result of a normal person, and then conducting by using an increase of an amount bound to the disease marker as an index,
(19) a disease marker comprising an antibody which recognizes CD81,
(20) the disease marker of the above item (19), which is used as a probe for diagnosing IBD,
(21) a method for diagnosing IBD, which comprises the following steps (a), (b) and (c):

(a) a step of binding a protein prepared from a biopsy of a subject to the disease marker of the above item (19) or (20),
(b) a step of measuring the protein derived from the biopsy bound to the disease marker using the disease marker as an index, and
(c) a step of judging the attack of IBD on the basis of the measurement results in (b),

(22) the method for diagnosing IBD of the above item (21), wherein the step of judging the attack of IBDdescribed in (c) is performed by comparing the measurement result of the subject with a measurement result of a normal person, and then conducting by using an increase of an amount bound to the disease marker as an index.

Herein disclosed is, as mentioned above, a screening system of a substance which reduces expression of a CD81 gene, a screening system of a substance which inhibits expression or a function (activity) of CD81, an agent for preventing, improving or treating IBD, containing a substance found in these screening systems as an active ingredient, and an agent for preventing, improving or treating IBD, containing an anti-CD81 antibody as an active ingredient. Further, it provides a disease marker of IBD, and a detection system of the diseases.

The invention is, as stated above, based on the findings that the expression of the CD81 gene in the IBD-pathogenic cells is increased in comparison to the IBD-non-pathogenic cells and the anti-CD81 antibody exhibits the therapeutic effect to inflammatory bowel disease model animals.

Accordingly, the CD81 gene, its expression products and derivatives thereof (for example, gene fragments and antibodies) are useful for screening substances which reduce expression of the CD81 gene or for screening substances which inhibit the CD81 expression or function (activity), and substances obtained by the screenings are effective as an agent for preventing, improving and treating inflammatory bowel diseases. Further, an anti-CD81 antibody or antisense nucleic acids (antisense nucleotides) of the CD81 gene are useful as an agent for preventing, improving or treating inflammatory bowel diseases.

Moreover, the CD81 gene, its expression products (proteins, polypeptides and oligopeptides) can be used effectively for elucidating, diagnosing, preventing and treating inflammatory bowel diseases (IBD), and medically and clinically profitable information or methods can be obtained by the use thereof. The detection of the expression of the gene or its expression products, or the detection of mutation of the gene or its expression abnormality can be utilized effectively for elucidation and diagnosis of inflammatory bowel diseases.

Best Mode for Carrying Out the Invention

In the present specification, amino acids, (poly)peptides, (poly) nucleotides and the like are indicated by abbreviations according to the regulation of IUPAC-IUB [IUPAC-IUB Communication on Biological Nomenclature, Eur. J. Biochem., 138: 9 (1984)], "Guideline for making specifications etc. including base sequences or amino acid sequences" (compiled by Japanese Patent Office) and common symbols in the field concerned.

In the specification, the "gene" or "DNA" is used such that it includes not only a double-stranded DNA but also respective single-stranded DNAs, a sense chain and an antisense chain constituting the double-stranded DNA. It is not particularly limited by the length thereof. Accordingly, the gene (DNA) in the specification includes, unless otherwise instructed, all of a double-stranded DNA including a human genomic DNA, a single-stranded DNA (plus chain) including a cDNA, a single-stranded DNA having a sequence complementary to the plus chain (complementary chain) and fragments thereof. Further, the "gene" or "DNA" includes not only a "gene" or "DNA" indicated by a specific base sequence (SEQ ID No: 1) but also a "gene" or "DNA" encoding proteins having an equal biological function to that of proteins thereby encoded [for example, homologous species (homologues, splice variants and the like), mutants and derivatives]. The "gene" or "DNA" encoding such homologous species, mutants or derivatives can include a "gene" or "DNA" having a base sequence to be hybridized with the complementary sequence of the specific base sequence of SEQ ID No: 1 under stringent conditions to be described later in (3-1).

For example, a gene encoding a homologue of a human protein can include genes of different organisms such as a mouse and a rat, corresponding to the human gene encoding the protein. These genes (homologues) can be identified by HomoloGene (http://www.ncbi.nlm.nih.gov/HomoloGene/). Specifically, a specific human base sequence is applied to BLAST (Proc. Natl. Acad. Sci. USA 90:5873-5877, 1993, http://www.ncbi.nlm.nih.gov/BLAST/) to obtain an accession number of a coincident (Score is the highest, E-value is 0 and Identity indicates 100%) sequence. This accession number is inputted in UniGene (http://www.ncbi.nlm.nih.gov/UniGene/), and the obtained UniGene Cluster ID (number indicated at Hs.) is inputted in HomoloGene. From the resulting list showing a gene homologue correlation of genes of different organisms and the human gene, the genes of different organisms such as a mouse and a rat can be selected as genes (homologues) corresponding to the human gene indicated by the specific base sequence.

The gene or DNA can include an expression control region, a code region, an exon or an intron without questioning a functional region.

When the term "CD81 gene" or "CD81 DNA" is used in the specification, it is used, unless otherwise instructed, such that it includes a human CD81 gene (DNA) indicated by the specific base sequence (SEQ ID No. 1) and a gene (DNA) encoding its homologous species, mutants and derivatives. Specifically, it includes the human CD81 gene (GenBank Accession No. NM_004356) described in SEQ ID No: 1, its mouse homologue and rat homologue, and so forth.

In the specification, the "polynucleotide" is used such that it includes both of an RNA and a DNA. The DNA includes all of a cDNA, a genomic DNA and a synthetic DNA. The RNA includes all of a total RNA, an mRNA, an rRNA and a synthetic RNA.

In the specification, the "protein" or the "(poly)peptide" includes not only a "protein" or a "(poly)peptide" indicated by a specific amino acid sequence (SEQ ID No: 2) but also its homologous species (homologues and splice variants), mutants, derivatives, mature substances, amino acid modified substances and the like so long as these are equal thereto in biological functions. Homologues here can include proteins of different organisms such as a mouse and a rat, corresponding to the human protein. They can be identified deductively from base sequences of genes identified by HomoloGene (http//www.ncbi.nlm.nih.gov/HomoloGene/). Mutants include alle mutants present in nature, mutants not present in nature and mutants having amino acid sequences which are modified by artificial deletion, substitution, addition or insertion. Further, the mutants can include those which are homologous to proteins or (poly)peptides without mutation by at least 70%, preferably 80%, more preferably 95%, further preferably 97%. The amino acid modified substances include amino acid modified substances present in nature and amino acid modified substances not present in nature. Specifically, amino acid phosphates can be mentioned.

When the term "CD81 protein" or only "CD81" is used in the specification, it is used, unless otherwise instructed, such that it includes human CD81 indicated by the specific amino acid sequence (SEQ ID No. 2) and its homologous species, mutants, derivatives, mature substances and amino acid modified substances. Specifically, human CD81 having an amino acid sequence described in SEQ ID No: 2 (GenBank Accession No. P18582), and its mouse homologue and rat homologue.

The "antibody" referred to in the specification include a polyclonal antibody, a monoclonal antibody, a chimera antibody, a single-stranded antibody and a part of the antibody having an antigen affinity, such as a Fab fragment or a fragment formed by a Fab expression library.

The "anti-CD81 antibody" in the specification includes an antibody which specifically recognizes CD81. Specifically, it includes an antibody capable of specifically recognizing an expression product (protein) of the CD81 gene (this is also referred to as "CD81" in the specification).

"IBD" in the specification is short for an inflammatory bowel disease. In Japan, it is called an inflammatory bowel disease. The inflammatory bowel disease includes, in view of the pathogenic state, ulcerative colitis (abbreviated as UC) and Crohn' s disease (abbreviated as CD).

The "disease marker" in the specification is one which is used directly or indirectly for diagnosing the presence or absence of attack of the inflammatory bowel disease (IBD), a degree of attack, the presence or absence of improvement and a degree of improvement, and for screening a candidate substance useful for preventing, improving or treating the inflammatory bowel disease (IBD). It includes polynucleotides, oligonucleotides or antibodies which can specifically recognize and bind to genes or proteins whose expression varies in vivo, especially in the large bowel tissue relative to the attack of the inflammatory bowel disease (IBD). These polynucleotides, oligonucleotides and antibodies can effectively be used, on the basis of the foregoing properties, as a probe for detecting the gene or proteins expressed in vivo, in tissues or in cells, and the oligonucleotides can effectively be used as a primer for amplifying the gene expressed in vivo.

Further, the " biopsy" to be diagnosed refers to a tissue in which the expression of the CD81 gene is increased with the inflammatory bowel disease (IBD). Specifically, it refers to a large bowel tissue and its surrounding tissues.

With respect to the CD81 gene (polynucleotide), its expression product (CD81) and its derivatives (anti-CD81 antibody and the like), the specific use thereof is described below.

(1) Screening method of a candidate agent

(1-1) Screening method using a gene expression level as an index

Herein disclosed is a method for screening a substance which reduces expression of CD81 gene.

The screening method includes the following steps (a), (b) and (c):

(a) a step of contacting a test substance with cells capable of expressing CD81 gene,
(b) a step of measuring an amount of the CD81 gene expression in the cells contacted with the test substance, and comparing the amount with an amount of the corresponding gene expression in control cells not contacted with the test substance, and
(c) a step of selecting the test substance reducing the amount of the CD81 gene expression on the basis of the comparison results in (b).

The cells used in the screening can include all culture cells, whether endogenous or exogenous, which express the CD81 gene. The expression of the CD81 gene can easily be recognized by detecting the gene expression thereof by the known northern blotting method or RT-PCR method. Specific examples of the cells used in the screening can include (A) intestinal tissue- or lymphatic tissue-derived cells isolated and prepared from an animal model of the inflammatory bowel disease (IBD), (B) intestinal tissue- or lymphatic tissue-derived cells treated or untreated with various stimulants, and (C) cells with any of the herein described genes introduced.

Here, as the animal model of IBD shown in (A), any of animal models known as IBD animal models may be used. Specific examples thereof can include spontaneously attacked IBD models (C3H/HeJBir mice, Cotton top tamarins and the like), chemical substance-induced models [dinitrochlorobenzene-induced models (rats), acetic acid models (rats), trinitrobenzenesulfonic acid- induced models (mice, rats and rabbits), dextran sulphate-induced models (mice, rats and hamsters), carrageenan-induced models (rats and guinea pigs), and indometacin-induced models (rats) and the like], transgenic/mutants (IL-2 knockout mice, IL-10 knockout mice, TCR knockout mice and the like) or cell transfer models (CD45RBhi-transfused SCID mice and the like). Preferable examples of the intestinal tissue-derived cells can include large bowel (colon)-derived primary culture cells.

The intestinal tissue-derived cells shown in (B) include preferably large bowel (colon)-derived cell strains. Specific examples thereof can include Caco-2 cells (derived from human colon gland cancer, ATCC No. HTB-37), HT-29 cells (derived from human colon gland cancer, ATCC No. HTB-38), COLO 205 cells (derived from human colon gland cancer, ATCC No. CCL-222) and the like. The lymphatic tissue-derived cells are preferably lymphocytes, monocytes, macrophages, neutrophils, eosinophils and cell strains thereof. Specific examples thereof can include RAW 264.7 cells (derived from mouse monocytes, ATCC No. TIB-71), U-937 cells (derived from human histocytic leukemia, ATCC No. CRL-1593.2), THP-1 cells (derived from human monocytes, ATCC No. TIB-202), Jurkat cells (derived from human T cell leukemia, ATCC No. TIB-152) and the like. Specific examples of the stimulant can include lipopolysaccharide (LPS), phorbol esters (PMA and the like), calcium ionophore, cytokines (IL-1, IL-6, IL-12, IL-18, TNF$\alpha$, IFN$\gamma$ and the like) and the like. It is known that cells are rendered in an activated state closer to an atmosphere of an inflammatory site.

Examples of the cells with the herein described gene introduced shown in (c) can include, in addition to the cells shown in (A) and (B), host cells ordinarily used in gene introduction, namely, mouse-derived cells and rat-derived cells such as L-929 (derived from a connective tissue, ATCC No. CCL-1), C127I (derived from a tissue of breast cancer, ATCC No. CRL-1616), Sp2/0-Ag14 (derived from myeloma, ATCC No. CRL-1581) and NIH3T3 (derived from a fetal tissue, ATCC No. CRL-1658), hamster-derived cells such as BHK-21 (derived from Syrian hamster kid renal tissue, ATCC No. CCL-10) and CHO-K1 (derived from a Chinese hamster oval, ATCC No. CCL-61), monkey-derived cells such as COS1 (derived from a renal tissue of an African green monkey, ATCC No. CRL-1650), CV1 (derived from a renal tissue of an African green monkey, ATCC No. CCL-70) and Vero (derived from a renal tissue of an African green monkey, Dainippon Pharmaceutical Co., Ltd.), human cells such as HeLa (derived from uterine cervix cancer, Dainippon Pharmaceutical Co., Ltd.) and 293 (derived from a fetal kidney, ATCC No. CRL-1573), and insect-derived cells such as Sf9 (Invitrogen Corporation) and Sf21 (Invitrogen Corporation).

Further, the cells used in the herein disclosed screening method also include a tissue which is an aggregation of cells, and the like.

The test substance (candidate substance) to be screened by the screening method is not limited, and it includes a nucleic acid (including an antisense nucleotide of the CD81 gene), a peptide, a protein, an organic compound, an inorganic compound and the like. The screening is specifically conducted by contacting these test compounds or samples (test samples) containing the same with the foregoing cells and/or tissues. Examples of such test samples include a cell extract, an expression product of a gene library, a synthetic low-molecular compound, a synthetic peptide, a natural compound and the like which contain the test substance, but are not limited to them.

In the herein described screening, the conditions under which the test substance is contacted with cells are not particularly limited. It is preferable to select culture conditions (temperatures, pH, a medium composition and the like) under which the cells are not dead and the CD81 gene can be expressed.

As will be demonstrated in Examples, the expression of the CD81 gene is specifically increased in the inflammatory bowel disease (IBD)-pathogenic cells in comparison to the inflammatory bowel disease (IBD)-non-pathogenic cells, and the anti-CD81 antibody exhibits a therapeutic effect to IBD model animals. From this finding, the expression of the CD81 gene is considered to have a causal relation with the inflammatory bowel diseases (IBD). Accordingly, the herein described screening method includes a method for searching a substance which reduces the expression of the CD81 gene (a substance which returns the expression level to the normal level) using the expression level of the CD81 gene as an index. The candidate substance having an alleviating/inhibiting activity of IBD (exhibiting an improving/treating effect to IBD) can be provided by this screening method.

That is, the herein described screening method is to provide the candidate substance which becomes an active ingredient of an agent for preventing, improving or treating IBD by searching the substance which reduces the expression of the CD81 gene.

The candidate substance can be selected specifically using, as an index, the fact that the expression level of the CD81 gene of the cell containing the test compound is lower than the very level of a test substance-free cell. When a cell requiring an expression-inducing substance is used in the expression of the CD81 gene, the selection can be conducted using, as an index, the fact that the expression induced in the presence of an expression-inducing substance [for example, lipopolysaccharide (LPS), phorbol esters (PMA and the like), calcium ionophore, cytokines (IL-1, IL-6, IL-12, IL-18, TNF$\alpha$ and IFN$\gamma$) and the like] is inhibited in the presence of the candidate substance, that is, the expression of the CD81 gene of the cells contacted with the candidate substance under expression-inducing conditions is decreased in comparison to that of the control cells (plus control) not contacted with the test substance in the presence of the expression-inducing substance.

The detection and the quantitative determination of the gene expression level in the herein disclosed screening method can be performed according to a known method such as a northern blotting method, an RT-PCR method or a method using a DNA chip as will be later described in (4-1) using the RNA prepared from the cells or the complementary polynucleotide transcribed from the RNA and the disease marker of the invention. Regarding the degree of change (inhibition or decrease) in the gene expression level as an index, it can be shown that the expression of the CD81 gene in the cells contacted with the test substance (candidate substance) is decreased (inhibited or reduced) by 10% or more, preferably 30% or more, especially preferably 50% or more in comparison to the expression amount in the control cells not contacted with the test substance (candidate substance).

The detection and the quantitative determination of the expression level of the CD81 gene can also be performed by measuring an activity of a protein derived from a marker gene such as a luciferase gene using a cell strain having introduced therein a fusion gene in which the marker gene is bound to the gene region controlling the expression of the CD81 gene (expression-controlling region). The herein disclosed screening method of the expression-inhibiting substance of the CD81 gene also includes a method for searching a target substance using the expression amount of the marker gene as an index. In this sense, the concept of the "CD81 gene" described herein includes the fusion gene of the expression-controlling region of the CD81 gene and the marker gene.

As the marker gene, a structural gene of an enzyme which catalyzes a luminous reaction or a color reaction is preferable. Specific examples thereof include, in addition to the luciferase gene, reporter genes such as a secretory alkaline phosphatase gene, a chloramphenicol·acetyl transferase gene, a β glucuronidase gene, a β galactosidase gene and an aequorin gene.

As the expression-controlling region of the CD81 gene here, a region of approximately 1 kb, preferably approximately 2 kb in the upstream of a transcription initiation site of the CD81 gene can be used. The expression-controlling region of the CD81 gene can be identified by, for example, a method comprising (i) a step of determining the 5'-terminal by an ordinary method such as a 5'-RACE method [performed using, for example, 5' full Race Core Kit (manufactured by Takara Shuzo)], an oligo-cap method or an S1 primer mapping; and (ii) a step of obtaining the 5'-upstream region with Genome Walker Kit (manufactured by Clontech) or the like and measuring a promoter activity of the resulting upstream region. Formation of the fusion gene and the measurement of the activity of the marker gene can be conducted by known methods.

The substances selected by the herein disclosed screening method can be regarded as a gene expression inhibitor of the CD81 gene. These substances inhibit attack or progression of large bowel tissue disorders by inhibiting the expression of the CD81 gene so that they become potent candidate substances of an agent for preventing, improving or treating the inflammatory bowel diseases (IBD).

(1-2) Screening method using an expression amount of protein as an index

Herein disclosed is a method for screening a substance which reduces the expression of CD81 (CD81 protein).

The screening method comprises the following steps (a), (b) and (c).

(a) a step of contacting a test substance with cells capable of expressing CD81,
(b) a step of measuring an expression amount of CD81 in the cells contacted with the test substance, and comparing the expression amount with an expression amount of the protein in control cells not contacted with the test substance, and
(c) a step of selecting the test substance reducing the expression amount of CD81 on the basis of the comparison results in (b).

The cells used in the screening can include all culture cells, whether endogenous or exogenous, which express the CD81 gene and have CD81 as an expression product. The expression of CD81 can easily be identified by detecting the protein as the gene product by the known western blotting method. Specific examples of the cells include the cells shown in (1-1) above. The cells also include cell membrane fractions, cytoplasm fractions, cell nucleus fractions thereof, and the like.

As shown in Examples, the expression of the CD81 gene is specifically increased in the IBD- pathogenic cells in comparison to the IBD-non-pathogenic cells, and the anti-CD81 antibody shows the therapeutic effect to IBD model animals. From these findings, the expression of CD81 is considered to have a causal relation with the inflammatory bowel diseases (IBD). Accordingly, the herein disclosed screening method includes a method for searching a substance which reduces the expression amount of CD81 (a substance which returns the expression level to the normal level) using the protein expression level of CD81 as an index. By this screening method, the candidate substance having an activity of alleviating/inhibiting IBD (exhibiting an improving/treating effect against IBD) can be provided.

That is, the herein disclosed screening method is to provide a candidate substance which becomes an active ingredient of an agent for preventing, improving or treating the inflammatory bowel diseases (IBD) by searching a substance which reduces the protein expression amount of CD81.

In case of specifically using cells expressing and producing CD81, the selection of the candidate substance can be performed using, as an index, the fact that the protein amount (level) of CD81 in cells containing the test substance (candidate substance) is reduced in comparison to the protein amount (level) in cells not containing the test substance (candidate substance). In case of using cells requiring the expression-inducing substance in the expression and production of CD81, the selection can be conducted using, as an index, the fact that the production of the protein induced by an expression-inducing substance [for example, lipopolysaccharide (LPS), phorbol esters (PMA and the like), calcium ionophore and cytokines (IL-1, IL-6, IL-12, IL-18, TNFα, IFNγ and the like] is inhibited by the presence of the test substance, that is, the expression of the CD81 gene of the cells contacted with the test substance in the presence of the expression-inducing substance is reduced in comparison to the control cells (plus control) not contacted with the test substance in the presence of the expression-inducing substance.

The production amount of CD81 in this herein disclosed screening method can be determined by a known method such as the western blotting method using an antibody (for example, an antibody which recognizes a human CD81 protein or its homologue), such as the herein described disease marker. The western blotting method can be performed by using the disease marker as a primary antibody, and then labeling with an antibody which is capable of binding to the primary antibody and labeled with a radioisotope such as $^{125}I$, a fluorescent substance or an enzyme such as horseradish peroxidase (HRP) as a secondary antibody, and measuring signals derived from these labeling substances with a radiation detector(BAS-1800II: manufactured by Fuji Photo Film.,Inc.,etc), a fluorescence detector or the like. Further, it is also possible that after using the disease marker as the primary antibody, detection is conducted with ECL Plus Western Blotting Detection System (manufactured by Amersham Pharmacia Biotech) according to its protocol and measurement is carried out with a multibioimager STORM860 (manufactured by Amersham Pharmacia Biotech).

The test substance (candidate substance) to be screened by the herein disclosed screening method is not limited. It includes a nucleic acid (including an antisense polynucleotide of the CD81 gene), a peptide, a protein, an organic compound, an inorganic compound and the like. The screening is specifically conducted by contacting these test substances or samples (test samples) containing the same with the foregoing cells or cell membrane fractions. Examples of the test samples include a cell extract, an expression product of a gene library, a synthetic low-molecular compound, a synthetic peptide, a natural compound and the like which contain the test substance. However, these are not critical.

(1-3) Screening method using a function'(activity) of a protein

Herein disclosed is a method for screening a substance which inhibits a function (activity) of CD81.

The screening method comprises the following steps (a), (b) and (c):

(a) a step of contacting a test substance with CD81,

(b) a step of measuring function (activity) of CD81 caused by the step in (a) above, and comparing the function (activity) with function (activity) of CD81 not contacted with the test substance, and

(c) a step of selecting the test substance inhibiting the function (activity) of CD81 on the basis of the comparison results in (b).

In the herein disclosed screening method, any method for measuring the function-activity based on a known function-activity of CD81 can be used. That is, a screening method in which a test substance is added to a known CD81 function-activity measuring system and a test substance which inhibits the known CD81 function-activity is selected as a candidate substance having an improving/treating effect against the inflammatory bowel diseases (IBD) falls under the category of the herein disclosed screening method.

The screening method can be conducted by contacting CD81-containing cells or cell fractions prepared from the cells with the test substance.

The cells used in the screening method can include cells, whether endogenous or exogenous, capable of expressing CD81. As the cells, the cells shown in (1-1) above, and the like can specifically be used. The cell fractions mean various fractions derived from the cells. Examples thereof include cell membrane fractions, cytoplasm fractions, cell nucleus fractions and the like.

CD81 used in the screening is a known protein, and it can be obtained by procedures, namely DNA cloning, construction of each plasmid, transfection into a host, incubation of a transformant cell and, as required, recovery of the protein from a culture. These procedures can be conducted according to a method known to a skilled person or a method described in documents [Molecular Cloning, T. Maniatis et al., CSH Laboratory (1983), DNA Cloning, DM. Glover, IRL PRESS (1985)] and the like.

Specifically, the CD81 protein can be obtained by producing a recombinant DNA (expression vector) capable of expressing a gene encoding CD81 in a desired host cell, introducing this DNA into a host cell for transformation, incubating the transformant and recovering the desired protein from the resulting culture.

As will be shown in Examples, the expression of the CD81 gene is specifically increased in the inflammatory bowel disease (IBD)-pathogenic cells in comparison to the IBD-non-pathogenic cells, and the anti-CD81 antibody exhibits the therapeutic effect to IBD model animals. From this finding, the increase in function (activity) of CD81 is considered to be related with the inflammatory bowel diseases (IBD). Accordingly, the herein disclosed screening method includes a method for searching a substance which inhibits a function (activity) of CD81 using the function (activity) of CD81 as an index. According to the screening method, the substance which inhibits the function (activity) of CD81 can be searched, and the candidate substance having the alleviating/inhibiting activity of the inflammatory bowel diseases (exhibiting the improving/treating effect against IBD) is thus provided.

That is, the herein disclosed screening method is to provide the candidate substance which becomes an active ingredient of the agent for preventing, improving or treating the inflammatory bowel diseases (IBD) by searching the substance which inhibits the function (activity) of CD81.

With respect to the selection of the candidate substance, the search for the substance which inhibits (reduces) the function (activity) of the CD81 protein can specifically be conducted using, as an index, the fact that the function (activity) of the protein obtained when contacting the CD81-containing cells or the cell fractions prepared from the cells with the test substance is reduced in comparison to the function (activity) of the protein of the test substance-free control cells or the cell fractions.

The test substance (candidate substance) to be screened by the herein disclosed screening method is not limited. Examples thereof include a nucleic acid, a peptide, a protein (including an anti-CD81 antibody), an organic compound, an inorganic compound and the like. The herein disclosed screening is specifically conducted by contacting these test substances or samples (test samples) containing the same with the aqueous solution, the cells or the cell fractions. Examples of the test samples include a cell extract, an expression product of a gene library, a synthetic low-molecular compound, a synthetic peptide, a natural compound and the like which contain the test substance. However, these are not critical.

Specific examples of the herein disclosed screening method based on the function (activity) of CD81 are as follows.

(A) Screening using an activity of inhibiting the binding as an index-1

CD81 is a cell surface molecule, and a fibronectin is known as its binding substance. Accordingly, on the basis of this known quality, screening of a test substance (candidate substance) which changes the function (activity) of CD81 can be conducted. That is, it can be conducted using the binding between CD81 as the protein and a substance which is bound to CD81, such as fibronectin (hereinafter sometimes referred to as a "CD81-binding substance"), as an index. In this case, a test substance which inhibits the binding between the CD81 protein and the CD81-binding substance can be selected as a candidate substance.

That is, the herein disclosed screening method based on the activity of inhibiting the binding includes a method comprising the following steps (a), (b) and (c):

(a) a step of contacting CD81 with a CD81-binding substance in the presence of a test substance,
(b) a step of measuring a binding amount obtained by the reaction and comparing the binding amount with a binding amount (control binding amount) obtained by conducting the step (a) in the absence of the test substance, and
(c) a step of selecting the test substance which changes the binding amount in comparison to the control binding amount on the basis of the results in (b).

In this case, examples of the inhibition or increase of the binding between the binding substance and CD81 include (i) the inhibition or increase of the binding between CD81 and the binding substance by binding to the binding substance and (ii) the inhibition or increase of the binding between CD81 and the binding substance by binding to CD81. However, it is not particularly limited so long as the binding is inhibited or increased.

(B) Screening using an activity of inhibiting the binding as an index-2

CD81 is also called TAPA-1. It has a function of differentiation, proliferation, activation or bonding of lymphocytes, and integrin is involved in its expression physiologically and functionally. In studies using an anti-CD81 antibody, it has been reported that integrin $\alpha4\beta1$ (VLA-4) is activated in B cells or IL-4 production from T cells antigen-presented in B cells is increased. Since integrin $\alpha4\beta1$ is bound to an extracellular substrate fibronectin, CD81 urges B cells to be bound to fibronectin of B cells [Behr S and et al. J. Exp Med 182:1191-1199 (1995)].

Accordingly, the inhibitory substance or candidate substance of inhibiting the function (activity) of CD81 can be screened by measuring the fibronectin-binding activity of integrin $\alpha4\beta1$-dependent B cells which is a known property of CD81. In this case, the candidate substance can be selected specifically, for example, when the fibronectin-binding activity of B cells provided by contacting the anti-CD81 antibody in the presence of the test substance (candidate substance) is reduced (inhibited) in comparison to the fibronectin-binding activity of B cells provided by contacting the anti-CD81 antibody in the absence of the test substance (candidate substance).

That is, the screening method based on the cell-binding activity of CD81 includes a method comprising the following steps (a), (b) and (c):

(a) a step of contacting CD81 expression cells with an anti-CD81 antibody in the presence of a test substance,
(b) a step of measuring a fibronectin-binding activity of B cells in (a) and comparing the binding activity value with a fibronectin-binding activity value (control activity value) obtained by contacting the cells with the anti-CD81 antibody in the absence of the test substance, and
(c) a step of selecting the test substance which reduces the activity value in comparison to the control activity value on the basis of the results in (b).

The screening methods in (A) and (B) which are the screening methods based on the function (activity) of CD81 can be performed using cells expressing CD81 or cell fractions thereof. Specific examples of the cells expressing CD81 include the cells shown in (1-1) above, cells in which CD81 is expressed in nature, transformant cells formed by introducing the gene encoding CD81 into cells, and the like. The CD81 expression cells used in the screening method in (B) may be B cells.
When cell membranes of the CD81 expression cells are used, for example, a hypotonic buffer is added to the cells, and the cells are hypotonically disrupted, followed by homogenization. Centrifugation is conducted to obtain a precipitate of a cell membrane fraction. This precipitate is suspected in a buffer to be able to obtain a cell membrane fraction containing a receptor. The resulting cell membrane fraction may be purified in a usual manner by a column having an antibody bound thereto or the like.
The transformant cells can be prepared by a method known to a skilled person according to a basic document such as Molecular Cloning 2nd Edt., Cold Spring Harbor Laboratory Press (1989). For example, a cDNA of CD81 is inserted into a known expression vector such as pCAGGS [Gene 108, 193-199 (1991)], pcDNA1.1 or pcDNA3.1 derivatives (Invitrogen). The resulting vector is then introduced into an appropriate host, and the transformant is incubated to be able to produce a transformant cell in which the protein corresponding to the DNA of CD81 introduced has been expressed. As the host, CHO cell, C127 cell, BHK21 cell, COS cell and the like which are generally widespread can be used. However, these are not critical, and yeasts, bacteria, insect cells and the like are also available.
As a method for introducing the expression vector having the z cDNA of CD81 into the host cell, any known methods for introduction of an expression vector into a host cell are available. Examples thereof include a calcium phosphate method [J. Virol., 52, 456-467 (1973)], a method using LT-1 (manufactured by Panvera), a method using gene introduction lipids (Lipofectamine and Lipofectin; manufactured by Gibco-BRL) and the like.
The protein may be used as such or by being labeled with an arbitrary labeling substance. Examples of the labeling substance here can include radioisotopes ($^{3}H$, $^{14}C$, $^{35}S$, $^{125}I$ and the like), fluorescent substances (Alexa Protein Labeling Kits of Molecular Probes, and the like), chemiluminescent substances (Chemiluminescence Labeling Kit of Assay Designs), biotin (EZ-Link Biotinylation Kits of Pierce and the like), marker proteins, peptide tags and the like. Examples of the marker proteins can include known marker proteins such as alkaline phosphatase (Cell, 63, 185-194, 1990), an Fc region of an antibody (GenBank accession number M87789) and HRP (horseradish peroxidase), and the like. Examples of the peptide tags can include known peptide tags such as Myc tag, His tag and FLAG tag.
The anti-CD81 antibody can be procured by purchasing a commercial anti-CD81 antibody (manufactured by Pharmingen).
The fibronectin-binding activity can easily be measured by referring to J Exp Med, 1995, 182: 1191-1199.
The test substance which is selectively obtained in this manner is a potent candidate of an agent for alleviating or inhibiting (preventing, improving or treating) the inflammatory bowel diseases (IBD)
The candidate substance selected by the herein disclosed screening method as described in (1-1) to (1-3) above may further be subjected to a pharmaceutical test and a safety test using IBD model animals such as (1) a spontaneously attacked IBD model (C3H/HeJBir mouse, Cotton top tamarins or the like), (2) a chemical substance-induced model [dinitrochlorobenzene-induced model (rat), an acetic acid model (rat), a trinitrobenzenesulfonic acid-induced model (mouse, rat or rabbit), a dextran sulfate-induced model (mouse, rat or hamster), a carrageenan-induced model (rat or guinea pig), an indometacin-induced model (rat) or the like, (3) a transgenic animal or mutant (IL-2 knockout mouse, IL-10 knockout mouse, TCR knockout mouse or the like) and (4) a cell transfer model (CD45RBhi transfer SCID mouse or the like), and a clinical test to IBD patients. A more practical IBD improving or treating agent can be obtained by performing these tests. The thus-selected substance can further be produced industrially by chemical synthesis, biological synthesis (fermentation) or gene manipulation on the basis of the results of its structural analysis.
All of the foregoing animal models are known to a skilled person, and animal models described in, for example, "In vivo models of Inflammation" compiled by D. W. Morgans (1999, Birkhauser Verlag Basel/Switzerland) can be used. Specifically, the spontaneously attacked IBD model (C3H/HeJBir mouse, Cotton top tamarins or the like) can be produced by the method described in Gastroenterology, vol. 107, pp. 1726-1735 (1994) or the like. The chemical substance (2,4,6-nitrobenzenesulfonic acid or the like is used)-induced model can be produced by the method described in Gastroenterology, vol. 96, pp. 795-803 (1989) or the like.

The screening methods described in (1-1) to (1-3) above can be used not only for selecting the candidate substance of the agent for improving or treating the inflammatory bowel diseases (IBD) but also for estimating and confirming whether or not the agent for preventing or treating IBD decreases the expression of the CD81 gene or whether or not it inhibits the expression or the function-activity of CD81. That is, the range of the screening method of the invention includes not only a method for searching the candidate substance but also a method for such estimation or confirmation.

(2) Agent for improving or treating the inflammatory bowel diseases (IBD)

(2-1) Anti-CD81 antibody to be used in accordance with the invention

The invention provides an anti-CD81 antibody effective for preventing, improving or treating IBD. The anti-CD81 antibody used in the invention is not particularly limited so long as it exhibits an effect of preventing, improving or treating the inflammatory bowel diseases (IBD).

The anti-CD81 antibody used in the invention can be obtained as a polyclonal or monoclonal antibody by a known method. As the anti-CD81 antibody used in the present invention, a monoclonal of, in particular, a mammalian origins, are preferred. The mammalian monoclonal antibody includes an antibody produced by a hybridoma and an antibody produced by a host transformed with an expression vector containing genetically engineered antibody genes. This antibody, via binding to CD81, remove CD81 expression cells, and thereby block or activate transmission of a biological activity of CD81 into cells.

The hybridoma producing an anti-CD81 antibody can be produced using basically a known technique describes bellow. That is, it can be produced by using CD81 as a sensitization antigen, immunizing it by a usual immunization method, fusing the resulting immune cells with known parent cells by a usual cell fusion method and screening monoclonal antibody-producing cells by a usual screening method.

Specifically, the anti-CD81 antibody may be produced as follows. For example, human CD81 used as a sensitization antigen for obtaining an antibody is formed by a known method.

It is advisable that the gene sequence of CD81 is inserted into a known expression vector system to transform an appropriate host cell and CD81 of the host cell surface is used as a sensitization antigen. CD81 expression cells, for example, all lymphatic cells may be used as a sensitization antigen.

Though mammals to be immunized with the sensitizing antigen are not specifically limited, they are preferably selected in consideration of their compatibility with the parent cell for use in cell fusion. They generally include, but not limited to, rodents such as mice, rats, hamsters and the like.

Immunization of animals with a sensitizing antigen is carried out using a known method. A general method, for example, involves the intraperitoneal or subcutaneous administration of a sensitizing antigen to the mammal. Specifically, a sensitizing antigen which has been diluted and suspended in an appropriate amount of phosphate buffered saline (PBS) or physiological saline etc. is mixed, as desired, with an appropriate amount of a common adjuvant, for example Freund's complete adjuvant. After being emulsified, it is preferably administered to a mammal for several times every 4 to 21 days. Alternatively a suitable carrier may be used at the time of immunization of the sensitizing antigen.

After immunization and the confirmation of the increase in the desired antibody levels in the serum, the immune cells are taken out from the mammal and are subjected to cell fusion, in which preferred immune cells include, in particular, the spleen cells.

The mammalian myeloma cells as the other parent cells which are subjected to cell fusion with the above-mentioned immune cells preferably include various known cell lines such as P3X63AgB. 653 (Kearney, J.F. et al., J. Immunol. (1979) 123: 1548-1550), P3X63Ag8U.1 (Current Topics in Microbiology and Immunology (1978) 81: 1-7), NS-1 (Kohler, G. and Milstein, C. , Eur. J. Immunol. (1976) 6: 511-519), MPC-11 (Margulies, D. H. et al., Cell (1976) 8: 405-415), SP2/0 (Shulman, M. et al., Nature (1978) 276: 269-270), FO (de St. Groth, S. F. et al., J. Immunol. Methods (1980) 35: 1-21), S194 (Trowbridge, I.S., J. Exp. Med. (1978) 148: 313-323), R210 (Galfre, G. et al., Nature (1979) 277: 131-133) and the like.

Cell fusion between the above immune cells and the myeloma cells may be essentially conducted in accordance with a known method such as is described in Milstein et al. (Kohler, G. and Milstein, C., Methods Enzymol. (1981) 73: 3-46) and the like.

More specifically, the above cell fusion is carried out in the conventional nutrient broth in the presence of, for example, a cell fusion accelerator. As the cell fusion accelerator, for example, polyethylene glycol (PEG), Sendai virus (HVJ) and the like may be used, and, in addition, an adjuvant such as dimethyl sulfoxide etc. may be added as desired to enhance efficiency of the fusion.

The preferred ratio of the immune cells and the myeloma cells to be used is, for example, 1 to 10 times more immune cells than the myeloma cells. Examples of culture media to be used for the above cell fusion include RPMI1640 medium and MEM culture medium suitable for the growth of the above myeloma cell lines, and the conventional culture medium used for this type of cell culture, and besides a serum supplement such as fetal calf serum (FCS) may be added.

In cell fusion, predetermined amounts of the above immune cells and the myeloma cells are mixed well in the above culture liquid, to which a PEG solution previously heated to about 37 °C, for example a PEG solution with a mean molecular weight of about 1000 to 6000, is added at a concentration of 30 to 60% (w/v) and mixed to obtain the desired fusion cells (hybridomas). Then by repeating the sequential addition of a suitable culture liquid and centrifugation to remove the supernatant, cell fusion agents etc., which are undesirable for the growth of the hybridoma, can be removed.

Said hybridoma is selected by culturing in the conventional selection medium, for example, the HAT culture medium (a culture liquid containing hypoxanthine, aminopterin, and thymidine). Culturing in said HAT culture medium is continued generally for a period of time sufficient to effect killing of the cells other than the desired hybridoma (non-fusion cells), generally several days to several weeks. The conventional limiting dilution method is conducted in which the hybridomas that produce the desired antibody are screened and monoclonally cloned.

In addition to obtaining the above hybridoma by immunizing an animal other than the human with an antigen, it is also possible to sensitize human lymphocytes in vitro with desired antigen or desired antigen-presenting cells, and the resulting sensitized B lymphocytes are fused with a human myeloma cell, for example U266, to obtain the desired human antibody having the activity of binding to desired antigen or desired antigen-presenting cells (see Japanese Post-examined Patent Publication (Kokoku) No. 1(1989)-59878). Furthermore, a transgenic animal having a repertoire of all human antibody genes is immunized with the antigen or the antigen-presenting cells to obtain the desired human antibody in the method described above (see InternationalPatent Application WO 93/12227, WO 92/03918, WO 94/02602, WO 94/25585, WO 96/34096 and WO 96/33735).

The monoclonal antibody-producing hybridomas thus constructed can be subcultured in the conventional culture liquid, or can be stored for a prolonged period of time in liquid nitrogen.

In order to obtain monoclonal antibodies from said hybridoma, there can be mentioned a method in which said hybridoma is cultured in the conventional method and the antibodies are obtained as the supernatant, or a method in which the hybridoma is administered to and grown in a mammal compatible with said hybridoma and the antibodies are obtained as the ascites. The former method is suitable for obtaining high-purity antibodies, whereas the latter is suitable for a large scale production of antibodies.

A recombinant antibody which was produced by the recombinant gene technology in which an antibody gene was cloned from the hybridoma and integrated into a suitable vector which was then introduced into a host can be used in the present invention as monoclonal antibody (see, for example, Borrebaeck C.A.K. and Larrick J.4V., THERAPEUTIC MONOCLONAL ANTIBODIES, published in the United Kingdom by MACMILLAN PUBLISHERS LTD. 1990).

Specifically, mRNA encoding the variable (V) region of the desired antibody is isolated from the hybridoma producing the antibody. The isolation of mRNA is conducted by preparing total RNA using, for example, a known method such as the guanidine ultracentrifuge method (Chirgwin, J.M. et al., Biochemistry (1979) 18, 5294-5299), the AGPC method (Chomczynski, P. et al., Anal. Biochem. (1987) 162, 156-159), and then mRNA is purified from the total RNA using the mRNA Purification kit (manufactured by Pharmacia) and the like. Alternatively, mRNA can be directly prepared using the Quick Prep mRNA Purification Kit (manufactured by Pharmacia).

cDNA of the V region of antibody may be synthesized from the mRNA thus obtained using a reverse transcriptase. cDNA may be synthesized using the AMV Reverse Transcriptase First-strand cDNA Synthesis Kit and the like. Alternatively, for the synthesis and amplification of cDNA, the 5'-Ampli FINDER RACE Kit (manufactured by Clontech) and the 5'-RACE method (Frohman, M.A. et al., Proc. Natl. Acad. Sci. U.S.A. (1988) 85, 8998-9002; Belyavsky, A. et al., Nucleic Acids Res. (1989) 17, 2919-2932) that employs polymerase chain reaction (PCR) may be used. The desired DNA fragment is purified from the PCR product obtained and may be ligated to vector DNA. Moreover, a recombinant vector is constructed therefrom and then is introduced into E. coli etc., from which colonies are selected to prepare the desired recombinant vector. The base sequence of the desired DNA may be confirmed by a known method such as the dideoxy method.

Once the DNA encoding the V region of the desired antibody has been obtained, it may be ligated to DNA encoding the constant region (C region) of the desired antibody, which is then integrated into an expression vector. Alternatively, the DNA encoding the V region of the antibody may be integrated into an expression vector which already contains DNA encoding the C region of the antibody.

In order to produce the antibody for use in the present invention, the antibody gene is integrated as described below into an expression vector so as to be expressed under the control of the expression regulatory region, for example an enhancer and/or a promoter.

Subsequently, the expression vector may be transformed into a host cell and the antibody can then be expressed therein.

In accordance with the present invention, artificially altered recombinant antibody such as chimeric antibody and humanized antibody can be used for the purpose of lowering heterologous antigenicity against humans. These altered antibody can be produced using known methods.

Chimeric antibody can be obtained by ligating the thus obtained DNA encoding the V region of antibody to DNA encoding the C region of human antibody, which is then integrated into an expression vector and introduced into a host for production of the antibody therein (see European Patent Application EP 125023, and International Patent Application WO 92/19759. Using this known method, chimeric antibody useful for the present invention can be obtained.

Humanized antibody which is also called reshaped human antibody has been made by grafting the complementarity determining regions (CDRs) of antibody of a mammal other than the human, for example mouse antibody, into the CDRs of a human antibody. The general recombinant DNA technology for preparation of such antibodies is also known (see European Patent Application EP 125023 and International Patent Application WO 92/19759.

Specifically, a DNA sequence which was designed to ligate the CDR of mouse antibody with the framework region (FR) of human antibody is synthesized from several divided oligonucleotides having sections overlapping with one another at the ends thereof by the PCR technique. The DNA thus obtained is ligated to the DNA encoding the C region of human antibody and then is integrated into an expression vector, which is introduced into a host for antibody production (see European Patent Application EP 239400 and International Patent Application WO 92-19759).

The FR of human antibody ligated through CDR, is selected so that the complementary determining region may form a favorable antigen binding site. When desired, amino acids in the framework region of the antibody variable region may be substituted so that the complementarity determining region of reshaped human antibody may form an appropriate antigen biding site (Sato, K. et al., Cancer Res. (1993) 53, 851-856).

For chimeric antibody or humanized antibody, the C region of human antibody is used. As the C region of human antibody, there can be mentioned Cγ, and Cγ1, Cγ2, Cγ3, or Cγ4, for example, can be used. The C region of human antibody may be modified to improve the stability of antibody or the production thereof.

Chimeric antibody consists of the variable region of antibody derived from a mammal other than the human and the C region derived from human antibody, whereas humanized antibody consists of the complementarity determining region of antibody derived from a mammal other than the human and the framework region (FR) and the C region of antibody derived from human antibody. Accordingly, antigenicity thereof in the human body has been reduced so that they are useful as the active ingredient of the therapeutic agents of the present invention.

Antibody genes constructed as described above may be expressed and obtained in a known method. In the case of mammalian cells, expression may be accomplished using a DNA in which a commonly used useful promoter, the antibody gene to be expressed, and the poly A signal at 3' downstream thereof have been operably linked or a vector containing said DNA. Examples of the promoter/enhancer include human cytomegalovirus immediate early promoter/enhancer.

Additionally, as the promoter/enhancer which can be used for expression of antibody for use in the present invention, there are viral promoters/enhancers such as retrovirus, polyoma virus, adenovirus, and simian virus 40 (SV40), and promoters/enhancers derived from mammalian cells such as human elongation factor 1α (HEF1α).

For example, expression may be readily accomplished by the method of Mulligan et al. (Nature(1979) 277, 108) when SV40 promoter/enhancer is used, or by the method of Mizushima et al. (Nucleic Acids Res. (1990) 18, 5322) when HEF1α promoter/enhancer is used.

In the case of E.coli, expression may be conducted by operably linking a commonly used useful promoter, a signal sequence for antibody secretion, and the antibody gene to be expressed, followed by expression thereof. As the promoter, for example, there can be mentioned lacz promoter and araB promoter. The method of Ward et al. (Nature (1989) 341, 544-546; FASEB J. (1992) 6, 2422-2427) may be used when lacz promoter is used, and the method of Better et al. (Science (1988) 240, 1041-1043) may be used when araB promoter is used.

As the signal sequence for antibody secretion, when produced in the periplasm of E. coli, the pelB signal sequence (Lei, S. P. et al., J. Bacteriol. (1987) 169, 4379) can be used. After separating the antibody produced in the periplasm, the structure of the antibody is appropriately refolded before use (see, for example, WO 96/30394).

As the origin of replication, there can be used those derived from SV40, polyoma virus, adenovirus, bovine papilloma virus (BPV) and the like. Furthermore, for the amplification of the gene copy number in the host cell system, expression vectors can include as selectable markers the aminoglycoside phosphotransferase (APH) gene, the thymidine kinase (TK) gene, E. coli xanthine guaninephosphoribosyl transferase (Ecogpt) gene, the dihydrofolate reductase (dhfr) gene and the like.

For the production of antibody for use in the present invention, any production system can be used. The production system of antibody preparation comprises the in vitro or the in vivo production system. As the in vitro production system, there can be mentioned a production system which employs eukaryotic cells and the production system which employs prokaryotic cells.

When the eukaryotic cells are used, there are the production systems which employ animal cells, plant cells, and fungal cells. Known animal cells include (1) mammalian cells such as CHO cells, COS cells, myeloma cells, baby hamster kidney (BHK) cells, HeLa cells, and Vero cells, (2) amphibian cells such as Xenopusoocytes, or (3) insect cells such as sf9, sf21, and Tn5. Known plant cells include, for example, those derived from Nicotiana tabacum, which is subjected to callus culture. Known fungal cells include yeasts such as the genus Saccharomyces, more specifically Saccharomyces cerevisiae, or filamentous fungi such as the genus Aspergillus, more specifically Aspergillus niger.

When the prokaryotic cells are used, there are the production systems which employ bacterial cells. Known bacterial cells include Escherichia coli (E.coli), and Bacillus subtilis.

By introducing via transformation the gene of the desired antibody into these cells and culturing the transformed cells in vitro, the antibody can be obtained. Culturing is conducted in the known methods. For example, as the culture liquid, DMEM, MEM, RPMI1640, and IMDM can be used, and serum supplements such as fetal calf serum (FCS) may be used in combination. In addition, antibodies may be produced in vivo by implanting cells into which the antibody gene has been introduced into the abdominal cavity of an animal and the like.

As in vivo production systems, there can be mentioned those which employ animals and those which employ plants. When animals are used, there are the production systems which employ mammals and insects.

As mammals, goats, pigs, sheep, mice, and cattle can be used (Vicki Glaser, SPECTRUM Biotechnology Applications, 1993). Also, as insects, silkworms can be used.

When plants are used, tabacco, for example, can be used.

Antibody genes are introduced into these animals or plants, and the antibodies are produced in such animals or plants, and recovered. For example, an antibody gene is inserted into the middle of the gene encoding protein which is inherently produced in the milk such as goat β casein to prepare fusion genes. DNA fragments containing the fusion gene into which the antibody gene has been inserted are injected into a goat embryo, and the embryo is introduced into a female goat. The desired antibody is obtained from the milk produced by the transgenic goat born to the goat who received the embryo or offsprings thereof. In order to increase the amount of milk containing the desired antibody produced by the transgenic goat, hormones may be given to the transgenic goat as appropriate. (Ebert, K.M. et al., Bio/Technology (1994) 12, 699-702).

When silkworms are used, baculovirus into which the desired antibody gene has been inserted is infected to the silkworm, and the desired antibody can be obtained from the body fluid of the silkworm (Maeda, S. et al., Nature. (1985) 315, 592-594). Moreover, when tabacco is used, the desired antibody gene is inserted into an expression vector for plants, for example pMON 530, and then the vector is introduced into a bacterium such as Agrobacterium tumefaciens. The bacterium is then infected to tabacco such as Nicotiana tabacum to obtain the desired antibody from the leaves of the tabacco (Julian, K. -C. Ma et al., Eur. J. Immunol. (1994) 24, 131-138).

When antibody is produced in in vitro or in vivo production systems, as described above, DNA encoding the heavy chain (H chain) or the light chain (L chain) of antibody may be separately integrated into an expression vector and the hosts are transformed simultaneously, or DNA encoding the H chain and the L chain may be integrated into a single expression vector and the host is transformed therewith (see International Patent Application WO 94-11523).

Antibodies for use in the present invention may be antibody fragments or modified versions thereof as long as they are preferably used. For example, as fragments of antibody, there may be mentioned Fab, F(ab')2, Fv or single-chain Fv (scFv) in which Fv's of H chain and L chain were ligated via a suitable linker. Specifically antibodies are treated with an enzyme, for example, papain or pepsin, to produce antibody fragments, or genes encoding these antibody fragments are constructed, and then introduced into an expression vector, which is expressed in a suitable host cell (see, for example, Co, M. S. et al., J. Immunol. (1994) 152, 2968- 2976; Better, M. and Horwitz, A.H., Methods in Enzymology (1989) 178, 476-496, Academic Press, Inc.; Plueckthun, A. and Skerra, A. , Methods in Enzymology (1989) 178, 476-496, Academic Press, Inc. ; Lamoyi, E., Methods in Enzymology (1986) 121, 652-663; Rousseaux, J. et al., Methods in Enzymology (1986) 121, 663-669; Bird, R. E. et al., TIBTECH (1991) 9, 132-137).

scFv can be obtained by ligating the V region of H chain and the V region of L chain of antibody. In the scFv, the V region of H chain and the V region of L chain are preferably ligated via a linker, preferably a peptide linker (Huston, J.S. et al., Proc. Natl. Acad. Sci. U. S.A. (1988) 85, 5879-5883). The V region of H chain and the V region of L chain in the scFv may be derived from any of the above-mentioned antibodies. As the peptide linker for ligating the V regions, any single-chain peptide comprising, for example, 12 - 19 amino acid residues may be used.

DNA encoding scFv can be obtained using DNA encoding the H chain or the H chain V region of the above antibody and DNA encoding the L chain or the L chain V region of the above antibody as the template by amplifying the portion of the DNA encoding the desired amino acid sequence among the above sequences by the PCR technique with the primer pair specifying the both ends thereof, and by further amplifying the combination of DNA encoding the peptide linker portion and the primer pair which defines that both ends of said DNA be ligated to the H chain and the L chain, respectively.

Once DNA encoding scFv are constructed, an expression vector containing them and a host transformed with said expression vector can be obtained by the conventional methods, and scFv can be obtained using the resultant host by the conventional methods.

These antibody fragments can be produced by obtaining the gene thereof in a similar manner to that mentioned above and by allowing it to be expressed in a host. "Antibody" as used in the claim of the present application encompasses these antibody fragments.

As modified antibodies, antibodies associated with various molecules such as polyethylene glycol (PEG) can be used. "Antibody" as used in the present application encompasses these modified antibodies. These modified antibodies can be obtained by chemically modifying the antibodies thus obtained. These methods have already been established in the art.

Antibodies produced and expressed as described above can be separated from the inside or outside of the host cell and then may be purified to homogeneity. Separation and purification of the antibody for use in the present invention may be accomplished by affinity chromatography. As the column used for such affinity chromatography, there can be mentioned Protein A column and Protein G column. Examples of the carriers used in the Protein A column are Hyper D, POROS, Sepharose F. F. and the like. Alternatively, methods for separation and purification conventionally used for proteins can be used without any limitation. Separation and purification of the antibody for use in the present invention may be accomplished by combining, as appropriate, chromatography other than the above-mentioned affinity chromatography, filtration, ultrafiltration, salting-out, dialysis and the like. Chromatography includes, for example, ion exchange chromatography, hydrophobic chromatography, gel-filtration and the like. These chromatographies can be applied into HPLC. Alternatively, reverse-phase chromatography can be used.

The concentration of antibody obtained in the above 2-1 can be determined by the measurement of absorbance or by the enzyme- linked immunosorbent assay (ELISA) and the like. Thus, when absorbance measurement is employed, the antibody for use in the present invention or a sample containing the antibody is appropriately diluted with PBS (-) and then the absorbance is measured at 280 nm, followed by calculation using the absorption coefficient of 1.35 OD at 1 mg/ml. When the ELISA method is used, measurement is conducted as follows. Thus, 100 ul of goat anti- human IgG (manufactured by TAGO) diluted to 1 ug/ml in 0.1 M bicarbonate buffer, pH 9.6, is added to a 96-well plate (manufactured by Nunc), and is incubated overnight at 4 °C to immobilize the antibody.

After blocking, 100 ul each of appropriately diluted antibody for use in the present invention or a sample containing the antibody, or 100 ul of human IgG (manufactured by CAPPEL) as the standard is added, and incubated at room temperature for 1 hour. After washing, 100 ul of 5000-fold diluted alkaline phosphatase-labeled anti-human IgG antibody (manufactured by BIO SOURCE) is added, and incubated at room temperature for 1 hour. After washing, the substrate solution is added and incubated, followed by the measurement of absorbance at405 nm using the MICROPLATE READER Model 3550 (manufactured by Bio-Rad) to calculate the concentration of the desired antibody.

The herein described anti-CD81 antibody can also be used as a screening tool for detecting the change in expression of CD81 described in (1) above or a diagnostic agent (disease marker) as will be later described in (3).

(2-2) Antisense polynucleotide

Herein disclosed is an antisense polynucleotide which contains a base sequence complementary or substantially complementary to the base sequence of CD81 or a part thereof, and which is effective for preventing, improving or treating IBD.

With respect to the antisense polynucleotide, any antisense polynucleotide is available so long as it contains a base sequence complementary or substantially complementary to the base sequence of the CD81 gene or a part thereof and has an activity capable of inhibiting the expression of the CD81 gene. It includes an antisense RNA, an antisense DNA and the like.

The substantially complementary base sequence here include a base sequence which is homologous to the base sequence complementary to the base sequence of the CD81 gene by approximately 70% or more, preferably approximately 80% or more, more preferably approximately 90% or more, most preferably approximately 95% or more, and the like. An antisense polynucleotide which is homologous to a complementary chain of a base sequence of a portion encoding an N-terminal site of the base sequence of the CD81 gene (for example, a base sequence near an initiation codon) by approximately 70% or more, preferably approximately 80% or more, more preferably approximately 90% or more, most preferably approximately 95% or more. Specifically, an antisense polynucleotide having a sequence complementary or substantially complementary to a base sequence described in SEQ ID No. 1 or a part thereof, and the like are mentioned.

The antisense polynucleotide comprises usually from 10 to 1,000 bases, preferably from 15 to 500 bases, more preferably from 16 to 30 bases. A phosphate residue of each nucleotide constituting the antisense DNA may be substituted with a chemically modified phosphate residue such as phosphorothioate, methyl phosphonate or phosphorodithionate. These antisense polynucleotides can be produced by a known DNA synthesizer or the like.

Such an antisense polynucleotide can be hybridized with an RNA of the CD81 gene to inhibit the synthesis or function of the RNA or to control the expression of the CD81 gene through the interaction with the RNA.

The herein disclosed antisense polynucleotide is useful for controlling the expression of the herein described protein gene in vivo or in vitro, and is also useful for treating diseases or the like. A 5'-terminal hairpin loop, a 5'-terminal 6-base pair repeat, a 5'-terminal non-translation region, a polypeptide translation initiation codon, a protein code region, an ORF translation termination codon, a 3'-terminal non-translation region, a 3'-terminal palindrome region, a 3'-terminal hairpin loop or the like of the protein gene may be selected as a preferable intended region. Any region in the protein gene may be selected. Regarding a relation between an objective nucleic acid and a polynucleotide at least partially complementary to the intended region, when the objective nucleic acid can be hybridized with the intended region, this objective nucleic acid can be said to be an "antisense" to the polynucleotide of the intended region.

Examples of the antisense polynucleotide include a polynucleotide containing 2-deoxy-D-ribose, a polynucleotide containing D-ribose, other types of polynucleotides which are N-glycosides of a purine or pyrimidine base, other polymers having a non-nucleotide skeleton (for example, a commercial protein nucleic acid and a synthetic sequence-specific nucleic acid polymer), other polymers containing a special bond (provided the polymers contain nucleotides having a sequence permitting a pair ring of bases or adhesion of bases found in a DNA or an RNA), and the like. These may be a double-stranded DNA, a single-stranded DNA, a double-stranded RNA, a single-stranded RNA and a DNA: RNA hybrid. Further, they may be an unmodified polynucleotide (or an unmodified oligonucleotide), a known modified substance such as a substance having a label known in the field concerned, a capped substance, a methylated substance, a substance in which one or more natural nucleotides are replaced with analogues, a substance with intramolecular nucleoside modification such as a substance having an uncharged bond (for example, methyl phosphonate, phosphotriester, phosphoramidate or carbamate), a substance having a charged bond or a sulfur-containing bond (for example, phosphorothioate or phosphorodithioate), such as a substance having a side chain group of a protein (for example, nuclease, nuclease inhibitor, toxin, antibody, signal peptide or poly-L-lysine) or a sugar (for example, monosaccharide), a substance containing an intercurrrent compound (for example, acridine or psolaren), a substance containing a chelating compound (for example, metal, radioactive metal, boron or oxidative metal), a substance containing an alkylating agent and a substance having a modified bond (for example, an $\alpha$-anomer-type nucleic acid). The "nucleoside", "nucleotide" and "nucleic acid" here may be not only those containing purine and pyrimidine bases but also those containing other modified heterocyclic bases. These modified substances may be substances containing methylated purine and pyrimidine, acylated purine and pyrimidine and other heterocycles. In the modified nucleotide and the modified nucleotide, the sugar moiety may be modified. For example, one or more hydroxyl groups may be substituted with a halogen, an aliphatic group or the like or may be converted to a functional group such as an ether or an amine. The herein disclosed antisense polynucleotide is an RNA, a DNA or a modified nucleic acid (RNA or DNA). Specific examples of the modified nucleic acid can include nucleic acid sulfur derivatives, nucleic acid thiophosphate derivatives, those having a resistance to degradation of polynucleoside amides or oligonucleoside amides, and the like.

The herein disclosed antisense polynucleotide can be designed, for example, as follows. That is, the antisense polynucleotide in a cell is rendered more stable, a cell permeability of the antisense polynucleotide is more increased, an affinity for a target sense chain is more increased, and a toxicity of an antisense polynucleoside which is toxic is reduced. A large number of such modifications are reported in, for example, Pharm Tech Japan, vol. 8, p. 247 or 395, 1992, and Antisense Research and Applications, CRC Press, 1993.

The herein disclosed antisense polynucleotide may be provided in a special form such as a liposome or a microsphere or in an addition form more adapted to gene therapy. Examples of the substance used in this addition form include a polycationic substance which acts to neutralize a charge of a phosphate group structure, such as polylysine, and a hydrophobic substance which enhances interaction with a cell membrane or increases incorporation of a nucleic acid, such as a lipid (for example, phospholipid or cholesterol). Examples of the preferable lipid to be added include cholesterol and its derivatives (for example, cholesteryl chloroformate and cholic acid). These can be added in the 3'-terminal or 5'-terminal of the nucleic acid and adhered via a base, a sugar or an intramolecular nucleoside bond. As the other group, a capping group located specifically in the 3'-terminal or 5'-terminal of the nucleic acid for inhibiting degradation with a nuclease such as exonuclease or RNase. As the capping group, protective groups of a hydroxyl group which are known in the field concerned, such as polyethylene glycol and tetraethylene glycol are mentioned. However, these are not critical. The inhibitory activity of the antisense polynucleotide can be measured by the herein disclosed screening method. The herein disclosed antisense polynucleotide may be a double-stranded one, as noted above, so long as it is bound to an RNA encoding CD81 to disrupt the RNA or inhibit its function. That is, a double-stranded RNA containing a part of the RNA encoding CD81 and an RNA complementary thereto, and the like are included in the herein disclosed antisense polynucleotide.

(2-3) Agent for preventing, improving or treating the inflammatory bowel diseases

Herein disclosed is an agent for preventing, improving or treating the inflammatory bowel diseases (IBD). In view of the new findings that CD81 is highly expressed in IBD-pathogenic cells and anti-CD81 antibody exhibits the therapeutic effect to the IBD model animals, the invention is based on the idea that the substance which inhibits CD81 gene expression, the substance which reduces the expression amount or function (activity) of CD81 and anti-CD81 antibody are effective for preventing, improving or treating the inflammatory bowel diseases.

Accordingly, the agent for preventing, improving and treating IBD, contains the substance which inhibits the expression of CD81 gene, the substance which inhibits the expression or function (activity) of CD81 or the anti-CD81 antibody as an active ingredient.

The substance which inhibits the expression of the CD81 gene or the substance which inhibits the expression or function (activity) of CD81, as the active ingredient, may be not only a substance which is selected by the foregoing screening method but also a substance which is industrially produced by a prescribed method on the basis of an information of a selected substance. Further, it may be subjected to a pharmaceutical test and a safety test using IBD model animals, and a clinical test to patients attacked with IBD. A more practical agent for improving or treating IBD can be obtained by performing these tests. The thus-selected substance can industrially be produced, on the basis of the results of its structural analysis, by chemical synthesis, biological synthesis (fermentation) or gene manipulation.

The substance which inhibits the CD81 gene expression or the substance which reduces the expression amount or function (activity) of CD81 can be used as such or prepared as a medical composition by being mixed with pharmaceutically acceptable carriers (including an excipient, an extender, a binder, a lubricant and the like) which are known per se or ordinary additives. The medical composition can be administered orally or parenterally according to the dosage forms (oral administration agents such as tablets, pills, capsules, powders, granules and syrup; parenteral administration agents such as injection solutions, drops, external preparations and suppositories) and the like. The dose varies depending on the type of the active ingredient, the route of administration, the age, weight and condition of administration object or patients, and the like, and it cannot absolutely be defined. However, regarding a dose per day, the medical composition can be administered at a dose of, usually from several milligrams to 2 g, preferably several tens of milligrams either once or in several divided portions daily.

An agent for preventing, improving or treating the inflammatory bowel diseases, containing an anti-CD81 antibody having an activity of inhibiting the function (activity) of the CD81 protein as an active ingredient is included herein.

Regarding the agent containing the anti-CD81 antibody as an active ingredient, both of the oral administration and the parenteral administration are possible. The parenteral administration is preferable. Specific examples thereof include an injection solution dosage form, a transnasal dosage form, a transpulmonary dosage form, a percutaneous dosage form and the like. Regarding examples of the injection solution dosage form, systemic or local administration can be conducted by intravenous injection, intramuscular injection, intraperitoneal injection, subcutaneous injection or the like.

The agent containing the anti-CD81 antibody as an active ingredient is itself administered as an agent formulated by a known manufacturing pharmaceutical method. For example, it can be used in the form of an injection solution of a sterile solution or suspension with water or other pharmaceutically acceptable liquids. Further, it is considered to be formulated by being properly mixed with pharmacologically acceptable carriers or media, such as sterile water, a physiological saline solution, an emulsifying agent, a suspending agent, a surfactant, a stabilizer, a vehicle and a preservative in the unit dosage form required for formulation, which is generally approved. The amount of the active ingredient in these pharmaceutical preparations is adjusted such that an appropriate volume in the prescribed range is obtained.

The sterile composition for injection can be formed according to ordinary formulation using a vehicle such as distilled water for injection. Examples of the aqueous solution for injection include a physiological saline solution and isotonic solutions containing glucose and other auxiliaries such as D-sorbitol, D-mannose, D-mannitol and sodium chloride. An appropriate solubilizer, for example, an alcohol such as ethanol, polyalcohol such as propylene glycol or polyethylene glycol, a nonionic surfactant such as polysorbate 80TM or HCO-50 may be used in combination.

Examples of oil include sesame oil and soybean oil, and benzyl benzoate or benzyl alcohol may be used in combination as a solubilizer. A buffering agent such as a phosphate buffer or a sodium acetate buffer, a soothing agent such as procaine hydrochloride, a stabilizer such as benzyl alcohol, phenol and an antioxidant may be incorporated. The thus-formed injection solution is usually filled in an appropriate ampule.

The dose can properly be selected depending on the age and condition of patients. For example, the dose can be selected in the range of from 0.0001 mg to 1,000 mg per kilogram of the body weight for one administration. Alternatively, the dose can be selected in the range of from 0.001 to 100,000 mg/body for a patient. However, the herein provided therapeutic agent is not limited by these doses.

Disclosed herein is the agent for preventing, improving or treating the inflammatory bowel diseases, containing the nucleotide or nucleotide derivatives for inhibiting the expression of the CD81 protein as an active ingredient. When the substance which inhibits the CD81 gene expression or the substance which reduces the expression amount or function (activity) of CD81 is encoded by a DNA, it is considered that the DNA is incorporated into a vector for gene therapy to conduct the gene therapy. When the active ingredient is an antisense nucleotide against the CD81 gene, the gene therapy can be conducted by using this antisense nucleotide as such or incorporating it into a vector. In these cases as well, the dose and administration method of the composition for gene therapy vary depending on the weight, age and condition of patients and the like, and can properly be selected by a skilled person.

The gene therapy using the antisense polynucleotide is described in detail below. The gene therapy can be performed, like this type of the ordinary gene therapy, by a method in which an antisense oligonucleotide or its chemically modified substance is administered directly into the body of a patient to control the expression of the objective gene or a method in which the antisense RNA is introduced into a target cell of a patient to inhibit the expression of the objective gene by the cell.

The antisense polynucleotide or its chemically modified substance can be bound to a sense chain mRNA in the cell to inhibit the expression of the objective gene, namely the expression of CD81 and thus inhibit the function (activity) of CD81.

In a method in which the antisense polynucleotide or its chemically modified substance is directly administered into a living body, it is advisable that the antisense polynucleotide or its chemically modified substance to be used has a length of, preferably from 10 to 1,000 bases, more preferably from 15 to 500 bases, most preferably from 16 to 30 bases. In the administration, the antisense oligonucleotide or its chemically modified substance can be formulated using a stabilizer, a buffer solution, a solvent and the like which are commonly used.

In the method for introducing the antisense RNA into a target cell of a patient, it is advisable that the antisense RNA to be used has a length of, preferably from 10 to 1,000 bases, more preferably from 15 to 500 bases, most preferably from 16 to 30 bases. Further, this method includes an in-vivo method in which the antisense gene is introduced into a cell in a living body and an ex-vivo method in which the antisense gene is introduced into a cell once taken out of the body and the very cell is returned into the body (refer to Nikkei Science, April 1994, pp. 20-45, Gekkan Yakuji, 36 (1), 23-48 (1994), Jikken Igaku Zokan, 12(15), all pages (1994), and the like). Of these, the in-vivo method is preferable, and it includes a viral introduction method (method using a recombinant virus) and a non-viral introduction method (refer to the foregoing documents).

The method using the recombinant virus includes methods in which the herein disclosed antisense polynucleotide is introduced into a living body by incorporating it into virus genomes of a retrovirus, associated a Lentivirus, an adenovirus, an adeno- virus, a herpes virus, a Sendai virus, a vaccinia virus, a poliovirus and a sindbis virus. Of these, the methods using a retrovirus, an adenovirus, an adeno-associated virus and the like are especially preferable. Examples of the non-viral introduction method include a liposome method, a lipofectin method and the like, and a liposome method is especially preferable. Other examples of the non-viral introduction method include a microinjection method, a calcium phosphate method, an electroporation method and the like.

The pharmaceutical composition for gene therapy contains, as an active ingredient, the foregoing antisense polynucleotide or its chemically modified substance, the recombinant virus containing the same, the infected cell having these viruses introduced therein or the like. The dosage form, the route of administration and the like in which the composition is administered to a patient can properly be determined depending on the disease and the symptom to be treated, and the like. For example, it can be administered intravenously, intraarterially, subcutaneously or intramuscularly in an appropriate dosage form such as an injection solution. Further, it can be administered and introduced directly in an intended disease site of a patient. When the in-vivo method is employed, the composition for gene therapy can be provided in a dosage form such as an injection solution containing the herein disclosed antisense polynucleotide or a dosage form in which a virus vector containing the antisense polynucleotide is embedded in a liposome or a membrane fusion liposome (Sendai virus (HVJ)-liposome or the like). These liposome dosage forms contain a suspending agent, a freezing agent, a centrifugal concentration freezing agent and the like. Further, the composition of gene therapy can be provided in a dosage form of a cell culture solution infected with the virus having introduced therein the vector containing the antisense polynucleotide of the invention. The dose of the active ingredient in the pharmaceutical preparations of these dosage forms can properly be adjusted depending on the degree of the disease to be treated, the age and weight of a patient and the like. In case of the antisense polynucleotide to the CD81 gene, it is advisable that the dose is usually from approximately 0.0001 to 100 mg, preferably from approximately 0.001 to 10 mg for an adult patient and administration is conducted once per several days or several months.

In case of the retrovirus vector containing the antisense polynucleotide, the dose can be selected from the range of from approximately $1 \times 10^3$ pfu to $1 \times 10^{15}$ pfu per kilogram of the patient's s weight daily in terms of a retrovirus potency. In case of the cell having the antisense polynucleotide introduced therein, it is advisable to conduct administration at a dose of from $1 \times 10^4$ cells/body to $1 \times 10^{15}$ cells/body.

Moreover, herein disclosed is (i) a double-stranded RNA containing a part of an RNA encoding the herein described protein and an RNA complementary thereto, (ii) an agent containing the double-stranded RNA, (iii) a ribozyme containing a part of an RNA encoding the herein described protein, (iv) an agent containing the ribozyme and (v) an expression vector containing a gene (DNA) encoding the ribozyme. Similarly to the antisense polynucleotide, a double-stranded RNA, a ribozyme and the like can disrupt an RNA transcribed from the DNA of the invention or inhibit its function. The double-stranded RNA, the ribozyme and the like which can inhibit the function of CD81 or the DNA encoding the same can be used as an agent for preventing or treating life style-related diseases, especially diabetes.

The double-stranded RNA can be produced by being designed on the basis of the sequence of the herein disclosed polynucleotide according to a known method (for example, Nature, vol. 411, p. 494, 2001). The ribozyme can be produced by being designed on the basis of the sequence of the herein disclosed polynucleotide according to a known method (for example, TRENDS in Molecular Medicine, vol. 7, P. 221, 2001). For example, it can be produced by replacing a part of a sequence of a known ribozyme with a part of the RNA encoding the herein disclosed protein. The part of the RNA encoding the protein includes a sequence near a consensus sequence NUX (in which N represents all bases and X represents bases other than G) which can be excised with a known ribozyme, and the like. When the double-stranded RNA or the ribozyme is used as the preventing or treating agent, it can be formulated and administered in the same manner as the antisense polynucleotide. The expression vector in (v) is used in the same manner as in a known gene therapy and as the foregoing preventing or treating agent.

(3) Disease marker of the inflammatory bowel diseases (IBD) and its application

(3-1) Polynucleotide

The herein disclosed CD81 gene is a known gene which is also called a TAPA-1 gene. For example, as a human CD81 gene, a polynucleotide described in SEQ ID No. 1 is known (GenBank Accession No. NM_004356), and a method for obtaining the same is also known as described in Prasad SS et al., Brain Res., 639, 73-84, 1994.

Starting with the finding that the expression of the CD81 gene is specifically increased in the inflammatory bowel disease (IBD)-pathogenic cells in comparison to the IBD-non-pathogenic cells, as stated above, it has been found herein that the presence or absence of the attack of IBD or the degree of its attack can specifically be detected by detecting the presence or absence of the expression of this gene or the degree of the expression and the diagnosis of the diseases can exactly be carried out.

The polynucleotide is, therefore, useful as a tool (disease marker) by which whether or not the subject is attacked by the inflammatory bowel diseases (IBD) or the degree of its attack can be diagnosed by detecting the presence or absence of the expression of the gene in the subject or its degree.

The polynucleotide is also useful as a screening tool (disease marker) for detecting the change in expression of the CD81 gene in the screening of the candidate substance useful for preventing, improving or treating the inflammatory bowel diseases (IBD) as described in (1-1) above.

The herein disclosed disease marker is characterized by comprising a polynucleotide having at least 15 continuous bases and/or a polynucleotide complementary thereto in the base sequence of CD81 gene.

Specifically, the herein disclosed disease marker can include one comprising a polynucleotide having at least 15 continuous bases and/or a polynucleotide complementary thereto in the base sequence of the CD81 gene described in SEQ ID No. 1.

The complementary polynucleotide (complementary chain, opposite chain) here means a polynucleotide having a complementary base relation with the full-length sequence of the polynucleotide comprising the base sequence of the CD81 gene or the partial sequence having a length of at least 15 continuous bases in the base sequence (here referred to also as "a plus chain" for convenience sake) on the basis of the base pair relation of A:T and G:C. However, this complementary chain may be not only one which has a completely complementary sequence to the base sequence of the intended plus chain but also one having such a complementary relation that it can be hybridized with the intended plus chain under stringent conditions. The stringent conditions here can be determined on the basis of a melting temperature (Tm) of a nucleic acid to which a complex or a probe is bound as described in Berger and Kimmel (1987, Guide to Molecular Cloning Techniques Methods in Enzymology, Vol. 152, Academic Press, San Diego CA). For example, as washing conditions after hybridization, conditions of "1 x SSC, 0.1% SDS, 37°C" can be mentioned. It is advisable that the complementary chain keeps the state hybridized with the intended plus chain even though washed under such conditions. More stringent conditions may involve washing under the conditions of around "0.5 x SSC, 0.1% SDS, 42°C", and further stringent conditions may involve washing conditions of around "0.1 x SSC, 0.1% SDS, 65°C", although it is not limited thereto. Specific examples of the complementary chain can include a chain comprising a base sequence having a completely complementary relation with the base sequence of the intended plus chain and a chain comprising a base sequence homologous to the plus chain by at least 90%, preferably 95%.

The polynucleotide on the plus chain side here can include not only a chain having the base sequence of the CD81 gene or its partial sequence but also a chain comprising a base sequence having a complementary relation with the base sequence of the complementary chain.

The polynucleotide of the plus chain and the polynucleotide of the complementary chain (opposite chain) may be used as a disease marker in the form of a single-stranded chain or a double-stranded chain respectively.

The herein disclosed disease marker of the inflammatory bowel diseases (IBD) may specifically comprise a polynucleotide comprising the base sequence (full-length sequence) of the CD81 gene or a polynucleotide comprising its complementary sequence. Further, a polynucleotide comprising the full-length sequence or the partial sequence of its complementary sequence is also available so long as it selectively (specifically) recognizes the herein described gene or the polynucleotide derived from this gene. In this case, regarding the partial sequence, the polynucleotide having a length of at least 15 continuous bases arbitrarily selected from the full-length sequence or the base sequence of its complementary sequence can be mentioned.

Here, "selectively (specifically) recognizes" means that the CD81 gene or the polynucleotide derived therefrom can specifically be detected in, for example, the northern blotting method and that the CD81 gene or the polynucleotide derived therefrom is specifically formed in the RT-PCR method. However, it is not critical. It is advisable that a skilled person can judge the detected product or the resulting product to be derived from these genes.

The herein disclosed disease marker can be designed using, for example, primer 3 (http:/www.genome.wi.mit.edu/cgi-bin/primer/primer 3.cgi) or vector NTI (manufactured by Infomax) on the basis of, for example, the base sequence of the CD81 gene indicated in SEQ ID No. 1. Specifically, a candidate sequence of a primer or a probe obtained by applying the base sequence of the CD81 gene to a software of primer 3 or vector NTI or a sequence containing at least a part of the sequence can be used as a primer or a probe.
As the herein disclosed disease marker, a disease marker having a length of at least 15 continuous bases as noted above is available. Specifically, the length thereof can properly be determined selectively depending on the use of the marker.

(3-2) Polynucleotide as a probe or a primer

The detection (diagnosis) of the inflammatory bowel diseases (IBD) is conducted by estimating the presence or absence of the expression or the expression level (expression amount) of the CD81 gene in a biopsy, especially a large bowel tissue of a subject. In this case, the herein disclosed disease marker can be used as a primer for specifically recognizing and amplifying an RNA formed by the expression of the foregoing gene or a polynucleotide derived therefrom or as a probe for specifically detecting the RNA or the polynucleotide derived therefrom.
When the herein disclosed disease marker is used as a primer in the detection (gene diagnosis) of the inflammatory bowel diseases (IBD), a disease marker having a base length of, usually from 15 bp to 100 bp, preferably from 15 bp to 50 bp, more preferably from 15 bp to 35 bp can be mentioned. When it is used as a detection probe, a disease marker having a base sequence of, usually from 15 bp to full length, preferably from 15 bp to 1 kb,more preferably from 100 bp to 1 kb can be mentioned.
The herein disclosed disease marker can be used as a primer or a probe in a usual manner in a known method for specifically detecting a specific gene, such as a northern blotting method, an RT-PCR method or an in-situ hybridization method. The presence or absence of the expression or the expression level of the CD81 gene in the inflammatory bowel diseases (IBD) can be estimated by this use.
As the sample to be measured, a total RNA which is prepared in a usual manner from a part of a large bowel tissue of a subject sampled by biopsy or the like or recovered from an intestinal wash liquid or the like, or various polynucleotides prepared on the basis of the RNA may be used according to the type of the detecting method used.
The gene expression level of the CD81 gene in the biopsy can be detected or determined using a DNA chip. In this case, the herein disclosed disease marker can be used as a probe of the DNA chip (for example, in case of Gene Chip Human Genome U95 A, B, C, D, E of Affymetrix, it is used as a polynucleotide probe having a length of 25 bp). This DNA chip is hybridized with the labeling DNA or RNA prepared on the basis of the RNA sampled from the biopsy, and a complex of the probe (disease marker of the invention) and the labeling DNA or RNA formed by the hybridization is detected using the label of the labeling DNA or RNA as an index, whereby the presence or absence or the expression level (expression amount) of the expression of the herein described gene in the biopsy can be estimated.
It is advisable that the DNA chip contains one or more herein disclosed disease markers which can be bound to the CD81 gene. The use of the DNA chip containing plural disease markers makes it possible to estimate the presence or absence of the expression or the expression level of plural genes at the same time on one biopsy.
The herein disclosed disease marker is useful for diagnosis and detection of the inflammatory bowel diseases (IBD)(diagnosis of the presence or absence of attack and a degree of attack). Specifically, the diagnosis of the inflammatory bowel diseases (IBD) using the disease marker can be conducted by judging a difference in gene expression level of the CD81 gene in the biopsy of a subject and the biopsy of a normal person. In this case, the difference in gene expression level includes not only a difference of whether or not the expression is present but also a case in which even though the expression is observed in both of the biopsy of the subject and the biopsy of the normal person, a difference in expression amount therebetween is twice or more, preferably three times or more.
Specifically, since the expression of the CD81 gene is significantly increased in the inflammatory bowel disease (IBD)-pathogenic cells in comparison to the inflammatory bowel disease (IBD)-non-pathogenic cells, the gene is expressed in the biopsy of the subject. When the expression amount is at least twice, preferably at least three times the expression amount of the biopsy of the normal person, the subject is presumed to be attacked by the inflammatory bowel disease (IBD).

(3-3) Antibody

Disclosed herein is an antibody which can specifically recognize an expression product (protein) of the CD81 gene (which is also referred to as "CD81" in the specification) as the disease marker of the inflammatory bowel diseases (IBD).
As the antibody, an antibody which can specifically recognize a CD81 protein having an amino acid sequence described in SEQ ID No. 2 can be mentioned. The antibodies described in (2-1) above are also available.

Starting with the finding that the expression of the CD81 gene is specifically increased in the inflammatory bowel disease (IBD)-pathogenic cells in comparison to the IBD-non-pathogenic cells, as stated earlier, it was found that the presence or absence of the attack of the inflammatory bowel diseases (IBD) or the degree of its attack can specifically be detected by detecting the presence or absence of the expression product of this gene or the degree of its expression and the diagnosis of the diseases can exactly be carried out.

The antibody is, therefore, useful as a tool (disease marker) by which whether or not the subject is attacked by the inflammatory bowel diseases (IBD) or the degree of the disease can be diagnosed by detecting the presence or absence of the expression of the protein in the subject or its degree.

The antibody is also useful as a screening tool (disease marker) for detecting the change in expression of CD81 in the screening of the candidate substance useful for preventing, improving or treating the inflammatory bowel diseases (IBD) as described in (1-2) above.

Human CD81 is also a known protein, and a method for obtaining the same is known as described in a document (BRENDAN J. CLASSON et al., J Exp Med, 169, 1497-1502, 1989).

The form of the antibody to be used in accordance with the invention is not particularly limited, and it may be a polyclonal antibody in which CD81 is an immunogen or its monoclonal antibody. Further, an antibody having an antigen affinity to a polypeptide comprising usually at least 8 continuous amino acids, preferably at least 15 continuous amino acids, more preferably at least 20 continuous amino acids of the amino acid sequence of the herein described protein is also included in the antibody to be used herein.

Processes for producing these antibodies are already well known, and the herein disclosed antibody can also be produced by these ordinary methods [Current Protocol in Molecular Biology, Chapter 11.12 - 11.13 (2000)]. Specifically, when the antibody is a polyclonal antibody, it is possible that non-human animals such as rabbits are immunized with CD81 expressed in E. coli or the like and purified by an ordinary method or with an oligopeptide having a part of the amino acid sequence of the herein described protein and formed by an ordinary method, whereby the antibody is obtained from the serum of the immunized animals by an ordinary method. Meanwhile, in case of the monoclonal antibody, it is possible that non-human animals such as mice are immunized with CD81 expressed in E. coli or the like and purified by an ordinary method or with an oligopeptide having a part of an amino acid sequence of the protein and the resulting spleen cells and myeloma cells are fused, whereby the antibody is obtained from among the resulting hybridoma cells (Current protocols in Molecular Biology edit. Ausubel et al. (1987) Publish. John Wiley and Sons, Section 11.4-11.11).

CD81 which is used as an immunogen in the production of the antibody is a known protein. As described in (1-3) above, for example, it can be obtained by procedures, namely, DNA cloning, construction of each plasmid, transfection into a host, incubation of a transformant and recovery of a protein from a culture on the basis of the sequence information (SEQ ID No: 1) provided herein. These procedures can be carried out according to a method known to a skilled person, a method described in documents [Molecular Cloning, T. Maniatis et al., CSH Laboratory (1983), DNA Cloning, DM. Glover, IRL PRESS (1985)] and the like.

The partial peptide of CD81 can also be produced by a general chemical synthesis method (peptide synthesis) according to an information of an amino acid sequence (SEQ ID No: 2) provided herein.

Herein disclosed CD81 includes not only a protein associated with each amino acid sequence indicated in SEQ ID No: 2 but also its homologue. The homologue can include a protein which comprises an amino acid sequence indicated in SEQ ID No: 2 in which one or more amino acids are deleted, substituted or added and which has an activity immunologically equal to that of the original protein before modification.

The protein having the equal immunological activity here can include a protein which induces a specific immune reaction in appropriate animals or cells thereof and which is capable of being specifically bound to an anti-CD81 antibody.

The mutation number or the mutation site of the amino acids in the protein are not particularly limited so long as the immunological activity thereof is maintained. The index of determining how and in what number the amino acid residues have to be substituted, inserted or deleted without losing the immunological activity can be found by a skilled person using a known computer program, for example, a DNA Star software. For example, the mutation number is typically within 10% of all amino acids, preferably within 5% of all amino acids, more preferably within 1% of all amino acids. The amino acids to be substituted are not particularly limited so long as the protein obtained after substitution maintains an equal immunological activity to that of CD81. In view of the structural retention of the protein, amino acids similar to amino acids before substitution in properties such as polarity, charge, solubility, hydrophobicity, hydrophilicity and amphipathic property of residues are preferable. For example, Ala, Val, Leu, Ile, Pro, Met, Phe and Trp are amino acids classified in non-polar amino acids; Gly, Ser, Thr, Cys, Tyr, Asn and Gln are amino acids classified in uncharged amino acids; Asp and Glu are amino acids classified in acidic amino acids; and Lys, Arg and His are amino acids classified in basic amino acids. Accordingly, amino acids belonging to the same group can properly be selected using these as an index.

The antibody to be used in accordance with the invention may be prepared using an oligopeptide having a part of the amino acid sequence of CD81. An oligo (poly) peptide used for production of this antibody is not required to have a functional bioactivity. However, it is advisable that the peptide has an equal immunogenicity to that of CD81. An oligo (poly) peptide having this immunogenicity and comprising at least 8 continuous amino acids, preferably at least 15 continuous amino acids, more preferably at least 20 continuous amino acids in the amino acid sequence of CD81 can be mentioned.

The production of the antibody against the oligo(poly)peptide may be conducted by increasing an immunological reaction with various adjuvants according to hosts. Examples of such adjuvants, though not limited, include Freunt adjuvant, a mineral gel such as aluminum hydroxide, lysolecithin, Pluronic polyol, polyanion, peptides, oil emulsions, surfactants such as keyhole limpet hemocyanine and dinitrophenol, and human adjuvants such as BCG (Bacille billie de Calmette-Guerin) and Corynebacterium parvum. Since the antibody to be used in accordance with the invention has a property of specifically binding to CD81, the protein expressed in a tissue of a subject can specifically be detected using this antibody. That is, the antibody is useful as a probe for detecting the presence or absence of the protein expression of CD81 in a tissue of a subject.

Specifically, CD81 can be detected by a known detection method such as a western blotting method or ELISA with the herein disclosed antibody employed as a probe in an ordinary manner using a tissue extract or protein prepared by an ordinary method from a part of a large bowel tissue of a subject which is collected by biopsy or the like or recovered from an intestinal wash liquid or the like.

In the diagnosis of the inflammatory bowel diseases (IBD), it is advisable to judge a difference in amount between CD81 in the biopsy of the subject and the protein in the normal biopsy. In this case, the difference in protein amount includes the presence/absence of the protein or the difference in protein amount that the protein amount is different by at least twice, preferably at least three times. Specifically, since the expression of the CD81 gene is increased in the IBD-inducing cells in comparison to the IBD-non-inducing cells, CD81 is present in the biopsy of the subject, and when the expression amount is judged to be at least twice, preferably at least three times the expression amount in the normal biopsy, the attack of the inflammatory bowel disease (IBD) is inferred.

(4) Detecting method (diagnosing method) of the inflammatory bowel diseases (IBD)

Herein disclosed is a detecting method (diagnosing method) of the inflammatory bowel diseases (IBD) using the foregoing disease marker.

Specifically, the herein disclosed detecting method (diagnosing method) comprises collecting a part of a large bowel tissue of a subject by biopsy or the like or recovering it from an intestinal wash liquid or the like, detecting a gene expression level of an inflammatory bowel disease-associated CD81 gene contained therein and a protein (CD81) derived from this gene, and measuring the expression amount or the protein amount to detect (diagnose) the presence or absence of the attack of the inflammatory bowel disease (IBD) or its degree. The herein disclosed detecting (diagnosing) method can also detect (diagnose) the presence or absence of the improvement of the disease or its degree in case of administering a therapeutic agent for improving the inflammatory bowel disease (IBD) to a patient of this disease.

The herein disclosed detecting method is a method comprising the following steps (a), (b) and (c):

(a) a step of contacting a biopsy of a subject with the herein described disease marker,
(b) a step of measuring a gene expression level of a CD81 gene in the biopsy or an amount of CD81 protein using the disease marker as an index, and
(c) a step of judging the attack of the inflammatory bowel disease (IBD) on the basis of the results in (b).

As the biopsy used here, a sample prepared from a biopsy(a large bowel tissue, its surrounding tissue or the like) of the subject can be mentioned. Specific examples thereof can include an RNA-containing sample prepared from the tissue, a sample containing a polynucleotide further formed therefrom, and a sample containing the protein prepared from the tissue. The sample containing such an RNA, polynucleotide or protein can be prepared by an ordinary method from a part of the large bowel tissue of the subject collected by biopsy or the like or recovered from an intestinal wash liquid or the like.

The herein disclosed diagnosing method can specifically be performed as follows according to the type of the biopsy to be measured.

(4-1) In case of using an RNA as a biopsy to be measured

When an RNA is used as a sample to be measured, the detection of the inflammatory bowel disease (IBD) can specifically be performed by a method comprising the following steps (a), (b) and (c):

(a) a step of binding a RNA prepared from a biopsy of a subject or a complementary polynucleotide transcribed therefrom to the herein described disease marker (a polynucleotide having at least 15 continuous bases in the base sequence of the CD81 gene and/or a polynucleotide complementary to the polynucleotide),

(b) a step of measuring the RNA derived from the biopsy or the complementary polynucleotide transcribed from the RNA wherein the RNA or the polynucleotide is bound to the disease marker, by using the disease marker as an index, and

(c) a step of judging the attack of the inflammatory bowel disease (IBD) on the basis of the measurement results in (b).

When an RNA is used as a substance to be measured, the herein disclosed detecting method (diagnosing method) is performed by detecting and measuring the expression level of the CD81 gene in the RNA. Specifically, it can be performed by conducting a known method such as a northern blotting method, an RT-PCR method, a DNA chip analysis method or an in-situ hybridization analysis method using the herein disclosed disease marker comprising the polynucleotide (a polynucleotide having at least 15 continuous bases in the base sequence of the CD81 gene and/or its complementary polynucleotide) as a primer or a probe.

In case of using the northern blotting method, the presence or absence of the expression of the CD81 gene in the RNA or its expression level can be detected and measured using the herein disclosed disease marker as a probe. Specifically, a method which comprises labeling the herein disclosed disease marker (complementary chain) with a radioisotope ($^{32}P$, $^{33}P$ or the like: RI), a fluorescent substance or the like, hybridizing the labeled marker with an RNA derived from a biopsy of a subject transferred onto a nylon membrane or the like in a usual manner and then detecting and measuring a signal derived from the labeling substance (RI or fluorescent substance) of the disease marker on the resulting double strand of the disease marker (DNA) and the RNA with a radiation detector (BAS-1800II, manufactured by Fuji Photofilm Co., Ltd.) or a fluorescence detector, can be mentioned. It is also possible to use a method which comprises labeling a disease marker (probe DNA) with AlkPhos Direct Labelling and Detection System (manufactured by Amersham Pharmacia Biotech) according to its protocol, hybridizing the labeled marker with an RNA derived from a biopsy of a subject, and then detecting and measuring a signal derived from the labeled disease marker with a multibioimager STORM860 (manufactured by Amersham

Pharmacia Biotech). In case of using the RT-PCR method, the presence or absence of the expression of the CD81 gene in the RNA or its expression level can be detected and measured using the herein disclosed disease marker as a primer. Specifically, a method which comprises preparing a cDNA from an RNA derived from a biopsy of a subject in a usual manner, hybridizing the cDNA with a pair of primers [a plus chain bound to the cDNA (- chain) and an opposite chain bound to the + chain] prepared from the herein disclosed disease marker so as to be able to amplify the region of the target CD81 gene with the cDNA as a template, conducting PCR in a usual manner and detecting the resulting amplified double-stranded DNA, can be mentioned. In the detection of the amplified double-stranded DNA, it is possible to use a method in which a labeled double-stranded DNA produced by conducting the PCR using a primer previously labeled with RI or a fluorescent substance is detected, a method in which the resulting double-stranded DNA is transferred onto a nylon membrane or the like in a usual manner and hybridized with a labeled disease marker as a probe to conduct the detection, or the like. The thus-produced labeled double-stranded DNA product can be measured with Agilent 2100 Bioanalyzer (manufactured by Yokokawa Analytical Systems). Moreover, it is also possible that an RT-PCR reaction solution is prepared with SYBR Green RT-PCR Reagents (manufactured by Applied Biosystems) according to its protocol and the reaction is conducted with ABI PRISM 7700 Sequence Detection System (manufactured by Applied Biosystems) to detect the reaction product.

In case of using the DNA chip analysis, a method which comprises preparing a DNA chip to which the herein disclosed disease marker is attached as a DNA probe (single-stranded or double-stranded), hybridizing it with a cRNA prepared from an RNA derived from a biopsy of a subject in a usual manner and binding the resulting double strand of the DNA and the cRNA to the labeling probe prepared from the herein disclosed disease maker to conduct detection, can be mentioned. As the DNA chip, a DNA chip capable of detecting and measuring the gene expression level of the CD81 gene is also available. As the DNA chip capable of detecting and measuring the expression level of the gene, Gene Chip Human Genome U95 A, B, C, D, E of Affymetrix can be mentioned. The detection and measurement of the gene expression level of the CD81 gene in the RNA of the subject will be described in detail in Examples.

(4-2) Use of a protein as a biopsy to be measured

When a protein is used as a substance to be measured, the method for detecting (diagnosing) the inflammatory bowel diseases (IBD) is performed by detecting CD81 in a biopsy and measuring its amount. Specifically, it can be performed by a method comprising the following steps (a), (b) and (c):

(a) a step of binding a protein prepared from a biopsy of a subject to the herein disclosed disease marker on an antibody (an antibody recognizing CD81),

(b) a step of measuring the protein derived from the biopsy bound to the disease marker using the disease marker as an index, and

(c) a step of judging the attack of the inflammatory bowel disease (IBD) on the basis of the measurement results in (b).

More specifically, a method for detecting and quantitatively determining CD81 by a known method such as a western blotting method using an antibody (an antibody recognizing CD81) as the herein disclosed disease marker on an antibody can be mentioned.

The western blotting method can be performed by conducting the labeling with the herein disclosed disease marker as a primary antibody and then with an antibody (bound to the primary antibody) labeled with a radioisotope such as $^{125}I$, a fluorescent substance or an enzyme such as horseradish peroxidase (HRP) and measuring signals derived from the radioisotope, the fluorescent substance or the like of the resulting labeled compound with a radiation meter (BAS-1800II: manufactured by Fuji Photofilm), a fluorescence detector or the like. Further, it is also possible that after using the herein disclosed disease marker as the primary antibody, detection is conducted with ECL Plus Western Blotting Detection System (manufactured by Amersham Pharmacia Biotech) according to its protocol and measurement is carried out with a multibioimager STORM860 (manufactured by Amersham Pharmacia Biotech.)

The function (activity) of CD81 to be measured in the foregoing method is already known, and has a prescribed interrelation with the amount and the function or activity of the protein. Accordingly, the herein disclosed detection (diagnosis) of the inflammatory bowel diseases (IBD) can also be conducted by measuring the function (activity) of the protein instead of measuring the amount of the protein. That is, also disclosed herein is a method for detecting (diagnosing) the inflammatory bowel diseases (IBD) in which the known function (activity) of CD81 as an index is measured and estimated according to the known method [specifically, refer to (1-3) above].

(4-3) Diagnosis of inflammatory bowel diseases (IBD)

The diagnosis of inflammatory bowel diseases (IBD) can be conducted by comparing the gene expression level of the CD81 gene in a biopsy(a large bowel tissue or the like) of a subject or an amount, a function or an activity (these are sometimes referred to as a "protein level" in combination) of the protein (CD81) which is the expression product of the gene with the gene expression level or the protein level in a normal biopsy (a large bowel tissue or the like) and judging the difference therebetween.

In this case, biopsy (samples containing an RNA or a protein) collected and prepared from a normal biopsy are required. These can be obtained by collecting biopsys (large bowel tissues or the like) of an inflammatory bowel disease (IBD)-unattacked person with a biopsy and the like or by recovering the same from an intestinal wash liquid or the like. Incidentally, "an inflammatory bowel disease (IBD)-unattacked person" here referred to means a person who is at least free from a subjective symptom of the inflammatory bowel disease and is preferably diagnosed to be unattacked by the inflammatory bowel disease (IBD) as a result of a detecting method such as endoscopy, barium enema or digestive organ biopsy. In the specification, "an inflammatory bowel disease (IBD)-unattacked person" is hereinafter sometimes referred to simply as a normal person.

The comparison in the gene expression level or the amount (level) of the protein between the biopsy of the subject and the normal biopsy (biopsy of the inflammatory bowel disease (IBD)-unattacked person) can be performed by conducting the measurement for the biopsy of the subject and the biopsy of the normal person simultaneously. When the measurement is not conducted simultaneously, an average value or a statistical medium value of gene expression levels of CD81 genes obtained by measuring plural (at least 2, preferably at least 3, more preferably at least 5) normal biopsys under uniform measurement conditions or of amounts (levels) of proteins (CD81) as expression products of these genes can be used in the comparison as the gene expression level or the protein amount of the normal person.

The judgment of whether or not a subject has the inflammatory bowel disease (IBD) can also be conducted using, as an index, the fact that the gene expression level of the CD81 gene or the protein level of its expression product in the biopsy of the subject is at least twice, preferably at least three times as high as that in the biopsy of the normal person.

When the gene expression level of the CD81 gene or the protein level of the subject is higher than that of the normal person, the subject is judged to be attacked by the inflammatory bowel disease (IBD) or is presumed to be attacked by this disease.

The invention is illustrated more specifically below by referring to Examples. However, the invention is not limited at all by these Examples.

EXAMPLES

Example 1

Preparation of inflammatory bowel disease (IBD) pathogenic cells

2,4,6-Trinitrobenzene sulfonic acid (TNBS)-induced IBD animal models can be induced according to a method described in Gastroenterology, vol. 96, pp. 795-803 (1989) or the like.

(1) Formation of a sensitizing solution

0.5 g of egg albumin (manufactured by Sigma) and 0.5 g of $K_2CO_3$ (manufactured by Nacalai Tesque) were dissolved in 25 ml of distilled water for injection (egg albumin solution). 0.5 g of TNBS (manufactured by Nacalai Tesque) was dissolved in 25 ml of 0.1 M $K_2CO_3$ (TNBS solution). The egg albumin solution and the TNBS solution were mixed, and the mixture was stirred overnight at room temperature. The stirred solution was dialyzed against 0.01 M $NaHCO_3$ (manufactured by Nacalai Tesque) through a dialyzer (Spectra/Por Membrane MWCO:10,000, manufactured by Spectrum Medical Industries Inc.). The dialyzate was subjected to protein quantitative determination with BCA Protein Assay Reagent (manufactured by Pierce). 2 mg/ml of the dialyzate was refrigerated as a sensitizing solution, and used by being dissolved when in use.

(2) TNBS-induced inflammatory bowel disease models

Complete Freunt adjuvant (CFA: manufactured by Difco) and 2 mg/ml of the sensitizing solution were subcutaneously injected (sensitized) into the back of a 4 to 6-week-old SJL/J mouse (purchased from Nippon Charles River) as an emulsion at a rate of 0.1 ml/head. On day 7 after the sensitization, 10 mg/ml of a TNBS 50% ethanol (manufactured by Nacalai Tesque) solution was intrarectally administered [0.2 ml/head was injected (challenged) by inserting a probe in a portion of up to 3 cm from the anus] under ether anesthesia. The symptom of colitis was scored according to the conditions of stool [normal stool (score: 0), loose stool (score:1) and diarrhea (score:2)]. The mouse attacked by colitis was brought to euthanasia on day 6 after the challenge, and the spleen and the mesenteric lymph node were extracted.

(3) Cell incubation

The mesenteric lymph node cells and the spleen cells treated with a hemolytic agent were inoculated in a culture flask (75T flask; manufactured by Iwaki Glass) in D-MEM (manufactured by Invitrogen) containing 10% fetal bovine serum (manufactured by BIOWHITTAKER) and 1% antibiotic-antimycotic with the cell number of $10^8$. The cells were incubated for 48 hours under the following three conditions. The cells were incubated in a $CO_2$ incubator under 5% $CO_2$ at 37°C. After the incubation, the cells were recovered, dissolved with TRIZOL (manufactured by LIFE TECHNOLOGIE), and stored at -80°C. Compound A is a compound having an activity of curing colitis by administering it to IBD animal models and an activity of inhibiting induction of IBD. It is a compound described in JP-A-2002-161084, namely 3-chloro-2-[1-(4-morpholin-4-ylphenyl)cyclobutyl]-4,5,6,7-tetrahyd ropyrazolo[1,5-a]pyrimidine.

A: no additive
B: addition of 0.2 μg/ml of Staphylococcal enterotoxin B (manufactured by Sigma)
C: addition of 0.2 μg/ml of Staphylococcal enterotoxin B (manufactured by Sigma) and 10 μM of compound A

The cells of conditions A and C are IBD-non-inducing cells which do not induce the symptom of colitis in normal mice even through transferred into the normal mice, whereas the cells of condition B are IBD-inducing cells which induce the symptom of colitis by being transferred into normal mice. That is, the causal factor of inducing the pathogenic state of colitis can be found by detecting the gene change of B cells relative to A and C cells.

Example 2

Analysis of gene expression of IBD- pathogenic cells and IBD-non-pathogenic cells with a DNA chip

(1) Preparation of a cDNA from a total RNA

A total RNA was prepared from the TRIZOL extract obtained in Example 1 according to the attached protocol. This total RNA was purified with RNeasy Mini Kit (manufactured by Qiagen), and the following cDNA synthesis was then conducted. The cDNA was synthesized from 500 ng of the total RNA using SuperScript Choice System (manufactured by Invitrogen), provided 100 pmol of T7-(24)dT primer (manufactured by Amersham Pharmacia Biotech) was used as a primer. The synthesized cDNA was purified with a DNA purification column (manufactured by Qiagen), and concentrated by ethanol precipitation. Then, a cRNA was synthesized by in-vitro transcription (MEGAscript T7 Kit: manufactured by Ambion) using the cDNA as a template. The cRNA was purified with RNeasy Mini Kit (manufactured by Qiagen), and a cDNA was synthesized using again SuperScript Choice System (manufactured by Invitrogen) on the basis of this cRNA. In the second cDNA synthesis, a random hexamer was used as a primer in the first strand (sense) synthesis, and T7-(24) dT primer as a primer in the second strand (antisense) synthesis (note: cRNA=antisense). The synthesized cDNA was purified by a DNA purification column, followed by measuring the concentration. By the foregoing procedures, each cDNA was obtained from each total RNA prepared in Example 1.

(2) Preparation of a labeled cRNA from a cDNA

DEPC-treated water was added to each cDNA in an amount corresponding to 200 ng to adjust the volume to 22 μL, and it was mixed with 4 μL of 10 x HY Reaction Buffer contained in BioArray High Yield RNA Transcript Labeling Kit (manufactured by ENZO), 4 μL of 10 x Biotin Labeled Ribonucleotides contained in this kit, 4 μL of 10 x DTT contained in this kit, 4 μL of 10 x RNase Inhibitor Mix contained in this kit and 2 μL of 20 x T7 RNA Polymerase contained in this kit. These were reacted at 37°C for 5 hours to prepare a labeled cRNA. After the reaction, 60 μL of DEPC-treated water was added to the reaction solution, and the resulting labeled cRNA was then purified with RNeasy Mini Kit (manufactured by Qiagen) according to the attached protocol.

(3) Fragmentation of a labeled cRNA

40 μL of a reaction solution obtained by adding 8 μL of 5 x Fragmentation Buffer [200 mM tris-acetate pH 8.1 (manufactured by Sigma), 500 mM potassium acetate (manufactured by Sigma) and 150 mM magnesium acetate (manufactured by Sigma)] to a solution containing 20 μg of each labeled cRNA was heated at 94°C for 35 minutes, and then put in ice. In this manner, the labeled cRNA was fragmented.

(4) Hybridization of a fragmented cRNA and a probe array

40 μL of each fragmented cRNA was mixed with 4 μL of 5 nM Control Oligo B2 (manufactured by Amersham), 4 μL of 100 x Control cRNA Cocktail, 40 μg of Herring sperm DNA (manufactured by Promega), 200 μg of Acetylated BSA (manufactured by Gibco-BRL), 200 μL of 2 x MES Hybridization Buffer [200 mM MES, 2M [$Na^+$], 40 mM EDTA and 0.02% Tween 20 (manufactured by Pierce), pH 6.5 to 6.7] and 144 μL of DEPC-treated water to obtain 400 μL of a hybri-cocktail. Each of the resulting hybri-cocktails was heated at 99°C for 5 minutes and further at 45°C for 5 minutes. After heating, centrifugation was conducted at room temperature and 14,000 rpm for 5 minutes to obtain a hybri-cocktail supernatant.

Meanwhile, Human Genome U95 Probe Array (manufactured by Affymetrix) filled with 1 x MES Hybridization Buffer was rotated within a hybri-oven at 45°C and 60 rpm for 10 minutes, and 1 x MES Hybridization Buffer was then removed to prepare a probe array. 200 μL of each of the resulting hybri-cocktail supernatant was added to the probe array, and rotated within the hybri-oven at 45°C and 60 rpm for 16 hours to obtain a probe array hybridized with the fragmented cRNA.

(5) Staining of the probe array

After the hybri-cocktail was recovered and removed from each of the resulting hybridized probe arrays, the probe array was filled with Non-Stringent Wash Buffer [6 x SSPE (dilution of 20 x SSPE (manufactured by Nacalai Tesque), 0.01% Tween 20 and 0.005% Antifoam 0-30 (manufactured by Sigma)]. Next, the probe array hybridized with the fragmented cRNA was set at a predetermined position of GeneChip Fluidics Station 400 (manufactured by Affymetrix) charged with Non-Stringent Wash Buffer and Stringent Wash Buffer (100 mM MES, 0.1 M NaCl and 0.01% Tween 20). Thereafter, the probe array was stained with a primary staining solution [10 μg/mL Streptavidin Phycoerythrin (SAPE) (manufactured by Molecular Probe), 2 mg/mL Acetylated BSA, 100 mM MES, 1 M NaCl (manufactured by Ambion), 0.05% Tween 20 and 0.005% Antifoam 0-30] and a secondary staining solution [100 μg/mL Goat IgG (manufactured by Sigma), 3 μg/mL Biotinylated Anti-Streptavidin antibody (manufactured by Vector Laboratories), 2 mg/mL Acetylated BSA, 100 mM MES, 1 M NaCl, 0.05% Tween 20 and 0.005% Antifoam 0-30] according to a staining protocol EuKGE-WS2.

(6) Scanning of a probe array and (7) Analysis of a gene expression amount

The stained probe arrays were subjected to HP GeneArray Scanner (manufactured by Affymetrix) to read staining patterns.

The expressions of the genes on the probe arrays were analyzed with GeneChip Workstation System (manufactured by Affymetrix) on the basis of the staining patterns. Then, normalization was conducted according to the analysis protocol, and the expression amount (average difference) of each probe (each gene) in each sample was calculated. Regarding the same probes, an average value of the gene expression amounts for each type of the sample was obtained, and the rate of change in expression amount between the respective types of the samples was then obtained.

Example 3

Analysis of change in expression

From the analytical results of the gene expressions by the DNA chip analysis conducted in Example 2, a probe set in which the expression was increased or decreased in Staphylococcal enterotoxin B (hereinafter sometimes abbreviated as "SEB")-containing inflammatory bowel disease (IBD)-pathogenic cells in comparison to SEB-free inflammatory bowel disease (IBD)-non- pathogenic cells was first selected.

From the probe set, 486 probes were selected in which the expression was decreased or increased in the IBD-non-pathogenic cells containing SEB and compound A in comparison to the IBD-pathogenic cells stimulated with SEB.

Subsequently, from these 486 probes, 56 probes which are an immunological inflammation-associated gene, a cell surface molecule and a growth factor gene were selected for selecting probes having a higher relation with the pathogenic state. Further, genes which are involved in the pathogenic state of IBD and have a function as a drug target by limitation to the change of a cell population contained in the analyzed cells were selected from these 56 probes.

Consequently, a total of ten genes, IL-17 gene, IL-9 gene, Lymphotoxin α gene, IL-10 preceptor β gene, DAP12 gene, TCR Vy4 gene, Integrin β1 gene, CD81 gene, CDw40 gene and paired-Ig-like receptor Al gene were selected (Table 1).

The IBD-non- pathogenic cells incubated without stimulation are increased in pathogenic state pathogenicity by SEB stimulation so as to become IBD- pathogenic cells. When compound A is added to the IBD-pathogenic cells, the pathogenic state pathogenicity is inhibited, and the cells become IBD-non- pathogenic cells. Accordingly, IL-17 gene, IL-9 gene, TCR Vγ4 gene, CD81 gene and CDw40 gene in which the gene expression amount is increased by SEB stimulation and is decreased by addition of compound A (Table 1) have been considered to be genes which can be applied as a disease marker that reflects the inflammatory bowel diseases. It has been considered that the attack of the inflammatory bowel diseases or the progression of the pathogenic state thereof can be inhibited or improved by decreasing the expression of these genes or the expression or the function (activity) of the proteins encoded by these genes. It has been further considered that Lymphotoxin α gene, IL-10 receptor β gene, DAP12 gene, Integrin β1 gene and paired-Ig-like receptor Al gene in which the gene expression amount is decreased by SEB stimulation and is increased by addition of compound A (Table 1) can also be applied as a disease marker that reflects the inflammatory bowel diseases, and that the attack of the inflammatory bowel diseases or the progression of the pathogenic state thereof can be inhibited or improved by increasing the expression of these genes or the expression or the function (activity) of proteins encoded by these genes. The table shows the results of the chip analysis of additive-free cells indicated by "Cont", SEB-incubated cells indicated by "SEB", and SEB and compound A-incubated cells indicated by "SEB+Compound".

Table 1

| Probe name | Gene name | Gene chip expression signal | | |
|---|---|---|---|---|
| | | Cont | SEB | SEB+Compound |
| 99349_at | IL-17 | 17.4 | 246.9 | 172.4 |
| 96574_at | IL-9 | 50.1 | 111.8 | 96.4 |
| 102630_s_at | Lymphotoxin α | 282.7 | 168.9 | 220.6 |
| 99491_at | IL-10Rβ | 1103.7 | 703.9 | 1000.9 |
| 100397_at | DAP12 | 1293.8 | 900.4 | 1144.2 |
| 102745_at | TCRV γ4 | 24.5 | 35.9 | 33.1 |
| 100124_r_at | Integrin β1 | 230.0 | 149.3 | 149.4 |
| 101495_at | CD81 | 179.8 | 210.4 | 195.1 |
| 92962_at | CDw40 | 38 | 72.8 | 63.9 |
| 100328_s_at | Paired-Ig-like receptor A1 | 917.7 | 705.7 | 791.2 |

Example 4

Function of compound A to intracellular IFN-γ

In interferon gamma [IFN-γ: J. Exp. Med 182:1281-1290 (1995)] already known as a pathogenic factor of the inflammatory bowel diseases in addition to the ten genes (IL-17 gene, IL-9 gene, Lymphotoxin α gene, IL-10 preceptor β gene, DAP12 gene, TCR Vγ4 gene, Integrin β1 gene, CD81 gene, CDw40 gene and paired-Ig-like receptor Al gene) selected in Example 3, the expression was increased in the SEB-containing IBD-inducing cells in comparison to the SEB-free IBD-non-pathogenic cells as a result of the DNA chip analysis of the gene expression. Further, it was a factor in which the expression was decreased in the IBD-non- pathogenic cells stimulated with SEB and compound A in comparison to the IBD-pathogenic cells stimulated with SEB.

On the assumption that a cell population on which compound A acts is the same as a cell population which inhibits an inflammatory bowel disease (IBD) pathogenicity by compound A, investigations were conducted to find an IBD-pathogenic cell population. Specifically, since intracellular IFN-γ is easy to detect, compound A acts on each cell population expressing any of the factors selected in Example 3, and it was examined which one of the factors selected in Example 3 was actually involved deeply in the IBD pathogenicity using the amount of intracellular IFN-γ as an index.

Intracellular IL-9 and intracellular Lymphotoxin α could not be detected for lack of an analytical reagent or the like. Regarding IL-17, an anti-IL-17 antibody was reported to worsen the symptom of the colitis model [Nihon Shokaki Kanren Gakkai Shukan (DDW-Japan 2003), Shokaki Byo Poster 337, Shiga Medical Collage, Shokaki Naika] and IL-17 was suggested to be a factor of improving colitis. Therefore, IL-17 was found to show an opposite function in search of an intended colitis-inducing factor. Accordingly, IL-17 was excluded in the analysis. Further, in Integrin β1 as a cell surface molecule, there are diverse expression cells, and it is judged that identification of a cell population on which compound A acts is impossible from the analytical results. Thus, Integrin β1 was excluded in the analysis. DAP 12 as a cell surface molecule could not be examined for lack of an analytical reagent or the like.

(1) Preparation and incubation of cells

Mesenteric lymph node cells and hemolysis reagent-treated spleen cells were prepared by the method of Example 1, and incubated in a culture flask (25T flask; manufactured by Iwaki Glass) in D-MEM (manufactured by Invitrogen) containing 10% fetal bovine serum (manufactured by BIOWHITTAKER) and 1% antibiotic-antimycotic with the cell number of 3.3 x $10^7$. The cells were incubated under the following two conditions for 48 hours. 4.5 ul BD Golgistop (manufactured by BD Biosciences) was added to the medium, and incubation was further conducted for 12 hours. The cell incubation was conducted in a $CO_2$ incubator under 5% $CO_2$ at 37°C. After the incubation, the cells were recovered, and IFN-γ in CD81-positive T cells was measured.

A: addition of 1 μg/ml Staphylococcal enterotoxin B (manufactured by Sigma)
B: addition of 1 μg/ml Staphylococcal enterotoxin B (manufactured by Sigma) and 10 μM of a compound of JP-A-2002-161084 (2) Measurement of intracellular IFN-γ

The cells were recovered, and suspended in a staining buffer (phosphate buffer containing 0.09% sodium azide and 1% bovine serum albumin). The viable cell number was counted with a trypan blue, and $10^6$ cells/100 ul were put into an Eppendorf tube, and 1 ul of an anti-CD16/CD32 antibody was added. The incubation was conducted at 4°C for 15 minutes. After the cells were washed, an FITC (fluoroisothiocyanate)-labeled antibody and a biotin-labeled antibody were added to the cells in an amount of 1 ug/$10^6$ cells (in 50 ul of a staining buffer), and the incubation was conducted at 4°C for 15 minutes. An anti-TCRV γδ antibody, an anti-CD4 antibody, an anti-TCRV β3 antibody, an anti-CD3 antibody, an anti-CD40 antibody or an anti-CD49d antibody was used as an FITC-labeled antibody. An anti-CD40L antibody, an anti-CD81 antibody or an anti-IL-10R antibody was used as a biotin-labeled antibody. After the cells were washed, a 1 ul Streptoavidin-labeled PerCP was added to the cells in an amount of 1 ug/$10^6$ cells (in 50 ul of a staining buffer), and incubation was conducted at 4°C for 15 minutes. After the washing, the cells were centrifuged, and 0.1 ml/tube of BD Cytofix/Cytoperm was added thereto. The incubation was conducted at 4°C for 20 minutes. The cells were washed twice with 1 ml/tube of 1 x BD Perm/Wash solution, and then to pellet. In 50 ul of 1 x BD Perm/Wash solution, a PE (phycoerythrin)-labeled anti-IFN-y antibody was added to the cells in an amount of 1 ug/$10^6$ cells, and the incubation was conducted at 4°C for 30 minutes. The resulting cells were washed twice with 1 ml/tube of 1 x BD Perm/Wash solution, and the supernatant was then discarded. The cells precipitated by centrifugation were loosened, and after replacement with a staining buffer, analysis was conducted with FACSCalibur (manufactured by BD Biosciences). The centrifugation was conducted at 4,000 r.p.m. and 4°C for 3 minutes (Centrifugal separator: TOMY MRX-152, rotor: TMA-6). Regarding a fluorescence intensity, FITC was measured with FL1, PE with FL2 and PerCP with FL3 respectively. As the biotin-labeled anti-IL-10R antibody, an antibody manufactured by Biolegend was used, while as all other antibodies, antibodies manufactured by BD Biosciences were used.

The results were shown in Table 2. A presence rate (%) of cell populations having various cell surface markers, a rate of intracellular IFN-γ-positive cells present in each cell population and reduced by compound A ("Compound-reacted cells" in the table) when a rate of intracellular IFN-γ-positive cells reduced by compound A is defined as 100 and a rate of compound-reacted cells for 1% of each cell population ("Compound-reacted cells / presence rate" in the table, frequency of presence of intracellular IFN-γ-positive cells reduced by compound A) were shown in Table 2. An anti-TCRV γδ antibody which is an obtainable reagent was used in the experiment for examining TCRV γ4-positive cells found in the change in gene expression, an anti-CD40 antibody which is an obtainable reagent was used in an experiment for examining CDw40-positive cells, and an anti-IL-10R antibody which is an obtainable reagent was used for examining IL-10Rβ-positive cells. Since IL-10-positive cells could not be detected as a clear cell population, the results were not provided.

Table 2

| Cell surface marker | Presence rate (%) | Compound-reacted cells (%) | Compound-reacted cells/presence rate |
|---|---|---|---|
| CD40 | 9.14 | 24.3 | 2.7 |
| CD49d | 16.4 | 39.6 | 2.4 |
| TCRV γδ | 5.39 | 23.8 | 4.4 |
| CD4 | 45.7 | 55.6 | 0.8 |
| TCRV β3 | 100 | 0 | 0 |
| CD3 | 92.1 | 103.4 | 1.1 |
| CD40L in CD3 | 3.94 | 3.3 | 0.8 |
| CD81 in CD3 | 4.67 | 51.7 | 11.1 |
| IL-10R in CD3 | * | * | * |

From the results in Table 2, it has been found that CD3 cells

(=T cells) are all of cell populations on which compound A acts (compound-reactive cells in the table are 103.4% in the cell surface marker CD3). It has been further found that CD81-positive cells of CD3-positive cells contain cell populations in which intracellular IFN-γ is most decreased by compound A at high frequency (the compound-reacted cells/presence rate in the table is 11.1 in the cell surface marker CD81 in CD3). Since compound A inhibits the IBD pathogenic state pathogenicity, the CD3-positive CD81-positive cells are cells by which the pathogenic state of the inflammatory bowel diseases is triggered. From these examination results, it has been clarified that CD81 is the pathogenic state-pathogenic cell marker in T cells and is a pathogenic factor which causes and progresses the pathogenic state of the inflammatory bowel diseases.

Example 5

Pharmacological effects of an anti-CD81 antibody to TNBS (2,4,6-trinitrobenzenesulfonic acid)-induced mouse colitis models

(1) Preparation of TNP-OVA

0.5 g of egg albumin (OVA: manufactured by Sigma) and 0.5 g of $K_2CO_3$ (manufactured by Nacalai Tesque) were dissolved in 25 ml of distilled water for injection (OVA solution). 0.5 g of 2,4,6-trinitrobenzenesulfonic acid (TNBS: manufactured by Nacalai Tesque) was dissolved in 25 ml of 0.1 M $K_2CO_3$ (TNBS solution). The OVA solution and the TNBS solution were mixed, and the mixture was stirred overnight at room temperature. The stirred solution was dialyzed against 0.01 M $NaHCO_3$ (manufactured by Nacalai Tesque) through a dialyzer (Spectra/Por Membrane MWCO:10,000, manufactured by Spectrum Medical Industries Inc.). The dialyzate was subjected to protein quantitative determination by BCA Protein Assay Reagent (manufactured by Pierce).

(2) Division, sensitization, challenge and administration

A 1:1 emulsion of complete Freunt adjuvant (CFA: manufactured by Difco Laboratories) and 2 mg/ml of TNP-OVA was subcutaneously injected (sensitized) into the back of a 5-week-old SJL/J mouse (SJL/JorllcoCrj, male, supplied by Nippon Charles River) at a rate of 0.1 ml/head. On day 7 after the sensitization, 10 mg/ml of a TNBS 50% ethanol solution was intrarectally administered (0.2 ml/head was injected (challenged) by inserting a probe in a portion of up to 3 cm from the anus) under ether anesthesia. On day 5 after the challenge, the mice were divided into three groups each consisting of 8 mice according to the weight and the score of the symptom.

As the groups, a group of administering 0.1 mg (0.2 ml)/head of an anti-CD81 antibody solution (Clone: 2F7, manufactured by Southernbiotech), a pathogenic state control group of administering 0.2 ml/head of a solvent, and a group of administering 200 mg/kg of sulfasalazine (SSZ: manufactured by Sigma). Regarding the administration, the drug was intraperitoneally administered to the antibody administration group and the pathogenic state control group on the division day and on day 2 after the division, and SSZ was orally administered continuously once daily from the division day. On day 5 after the division, the experiment was completed. The SSZ solution was suspended in a 0.5% methylcellulose (manufactured by Nacalai Tesque).

(3) Weight measurement and evaluation with symptom scores

The symptom of colitis from the division day to day 5 after the division was observed. The symptom of colitis was scored according to the conditions of stool [normal stool (score:0), loose stool (score:1) and diarrhea (score:2)]. Dead individuals were excluded from the results. With respect to the statistical analysis, round-robin multi-group comparison of the pathogenic state control group, the anti-CD81 antibody administration group and the SSZ administration group was conducted by the Steel test (*; $0.01 < p < 0.05$, **; $p < 0.01$).

(4) Measurement of a length of a large bowel

On the final day of the experiment, after euthanasia with a high concentration of $CO_2$, a large bowel was extracted, and the length thereof was measured. Regarding the length of the large bowel in each group, round-robin statistical analysis in the pathogenic state control group, the anti-CD81 antibody group and the SSZ administration group was conducted by a Dunnet test after a Bartlett test (*; $0.01 < p < 0.05$, **; $p < 0.01$).

The results were shown in Table 3. By the anti-CD81 antibody, the symptom of colitis of the TNBS-induced colitis mouse and the shortening of the intestinal length were improved equally to those by SSZ. These results have revealed that the anti-CD81 antibody can be an agent for preventing, improving or treating the inflammatory bowel diseases.

Table 3

| Group | Symptom of colitis (S.D) | Intestinal Length mm(S.D) |
|---|---|---|
| Pathogenic state control | 1.75 (0.46) | 87.8 (10.3) |
| Anti-CD81 antibody administration | 0.75 (0.76)* | 98.6 (6.9)* |
| SSZ administration | 0.71 (0.71)* | 103.1 (8.3)** |

Example 6

Screening of a CD81 gene expression inhibitor

Jurkat cells (derived from human T cell lymphoma), NC-37 cells (derived from human peripheral lymphoblasts, ATCC No. CCL-214), CCRF-SB cells (derived from human acute lymphoblast leukemia B cells, ATCC No. CCL-120), CCRF-CEM cells (derived from human acute lymphoblast leukemia T cells, ATCC No. CCL-119), MOLT-3 cells (derived from human acute lymphoblast leukemia T cells, ATCC No. CRL-1552), CCRF-HSB-2 cells (derived from human acute lymphoblast leukemia T cells, ATCC No. CCL-120.1), IM-9 cells (derived from human peripheral lymphoblasts, ATCC No. CCL-159), EB-3 cells (derived from human Burkitt lymphoma, ATCC No. CCL-85), CA46 cells (derived from human Burkitt lymphoma, ATCC No. CRL-1648), Daudi cells (derived from human Burkitt lymphoma, ATCC No. CCL-213), Namalwa cells (derived from human Burkitt lymphoma, ATCC No. CRL-1432), Raji cells (derived from human Burkitt lymphoma, ATCC No. CCL-86), Ramos (RA1) cells (derived from human Burkitt lymphoma, ATCC No. CCL-1596), RPMI 1788 cells (derived from human peripheral lymphocytes, ATCC No. CCL-156), RPMI 6666 cells (derived from human Hodgkin disease lymphoblasts, ATCC No. CCL-113), U-937 cells (derived from human histiocytic lymphoma, ATCC No. CRL-1593), B-ATL1 cells (derived from human adult T cell leukemia patient B cells), B-ATL2 cells (derived from human adult T cell leukemia patient B cells), B-ATL7 cells (derived from human adult T cell leukemia patient B cells), B-ATL8 cells (derived from human adult T cell leukemia patient B cells), OKM-2T cells (derived from human adult T cell leukemia patient T cells) or OKM-3T cells (derived from human adult T cell leukemia patient T cells) (all these can be purchased from Dainippon Pharmaceutical Co., Ltd.), or human peripheral lymphocytes are incubated in a Dulbecco's Modified Eagle medium containing 10% inactivated fetal bovine serum, 2 mM glutamine, 50 IU/ml penicillin and 50 mg/ml streptomycin at 37°C and a $CO_2$ concentration of 5%.

The counted cells are inoculated in a 24-well tissue culture plate at a concentration of $0.6\text{-}1.2 \times 10^5$ cells/cm$^2$,

and cultured at 37°C and a $CO_2$ concentration of 5%. A test substance-containing solution (solution containing the test substance at a concentration of 100 μM, 10 μM or 1 μM) is added to these cells in the presence of a stimulant [phorbol 12-myristate 13 acetate, concanavalin, lipopolysaccharide, cytokines (interferons, tumor necrosis factor and interleukins), an anti-T cell surface antigen antibody, an anti-B cell surface antigen antibody or butyrate). In this case, test substance-free cells are also incubated in the same manner as a control experiment (control).

Gene expression amounts are analyzed by the method described in Example 2 using RNAs extracted from these culture cells. Consequently, a test substance which decreases (inhibits or reduces) the expression of the CD81 gene by 10% or more, preferably 30% or more, especially preferably 50% or more in comparison to the expression amount in the control cells which are not contacted with a test substance (candidate substance) is selected as a candidate compound which alleviates or controls (improves or treats) the inflammatory bowel diseases (IBD).

Example 7

Screening of a CD81 function (activity) inhibitor For coating a 24-well culture plate with fibronectin (Sigma), fibronectin adjusted to a concentration of 10 μg/ml with PBS is adsorbed on a 24-well culture plate (manufactured by Falcon Labware) at 37°C for 3 hours, and then washed three times with PBS. Human B cells [Daudi cells (derived from human Burkitt lymphoma, ATCC No. CCL-213, manufactured by Dainippon Pharmaceutical Co., Ltd.), Namalwa cells (derived from human Burkitt lymphoma, ATCC No. CRL-1432, manufactured by Dainippon Pharmaceutical Co., Ltd.), Raji cells (derived from human Burkitt lymphoma, ATCC No. CCL-86, manufactured by Dainippon Pharmaceutical Co., Ltd.), and Ramos (RA1) cells (derived from human Burkitt lymphoma, ATCC No. CCL-1596, manufactured by Dainippon Pharmaceutical Co., Ltd.) reacted with the anti-CD81 antibody are washed, then inoculated in a plate at a concentration of 1 x $10^5$ cells/well, incubated at 37°C for 20 minutes, washed three times with PBS, and immobilized with 3% glutaraldehyde. The cells bound to the plate are counted by observation with a microscope of approximately 400 x magnification. The anti-CD81 antibody can be obtained as a commercial product (Pharmingen or the like).

The rate of inhibition of the B cell fibronectin binding activity by the test substance is expressed by the following formula:

{[Number of cells in the absence of a test substance) - (Number of cells in the presence of a test substance)]/(number of cells in the absence of a test substance)} x 100%

A test substance added to a system in which the rate of inhibition of the B cell fibronectin binding activity is changed by 10% or more, preferably 30% or more, especially preferably 50% or more is selected as a candidate compound of alleviating or controlling (improving or treating) the inflammatory bowel diseases (IBD).

INDUSTRIAL APPLICABILITY

The invention has clarified that the expression of the CD81 gene is specifically increased in the inflammatory bowel disease (IBD)-pathogenic cells in comparison to the IBD-non-pathogenic cells and that the anti-CD81 antibody is effective for treating the inflammatory bowel diseases.

An agent containing the anti-CD81 antibody as an active ingredient can be used in inflammatory bowel disease patients as a therapeutic agent of the inflammatory bowel diseases. Further, owing to a relation of the expression of the CD81 gene and the inflammatory bowel diseases (IBD), a candidate agent capable of becoming a therapeutic agent of the inflammatory bowel diseases can be screened and selected using, as an index, the inhibition of the expression of the CD81 gene or the inhibition of the expression or the function (activity) of the protein encoded by the CD81 gene.

Further, the CD81 gene is useful as a marker gene (probe or primer) used in the gene diagnosis of the inflammatory bowel diseases. Such a marker gene makes it possible to clarify whether or not the disease is the inflammatory bowel disease (improve the diagnostic precision), whereby more appropriate treatment can be applied.

**Claims**

1. Use of anti-CD81 antibody for the preparation of a pharmaceutical composition for preventing, improving or treating inflammatory bowel disease (IBD).

2. The use according to claim 1, wherein the CD81 is a mammal CD81.

3. The use according to claim 1 or 2, wherein the anti-CD81 antibody is a monoclonal antibody.

4. The use according to any one of claims 1 to 3, wherein said inflammatory bowel disease is ulcerative colitis.

5. The use according to any one of claims 1 to 3, wherein said inflammatory bowel disease is Crohn's disease.

6. An anti-CD81 antibody for use in preventing, improving or treating inflammatory bowel disease (IBD).

7. The anti-CD81 antibody for use of claim 6, wherein the CD81 is a mammal CD81.

8. The anti-CD81 antibody for use of claim 6 or 7, wherein said antibody is a monoclonal antibody.

9. The anti-CD81 antibody for use of any one of claims 6 to 8, wherein said inflammatory bowel disease is ulcerative colitis.

10. The anti-CD81 antibody for use of any one of claims 6 to 8, wherein said inflammatory bowel disease is Crohn's disease.

**Patentansprüche**

1. Verwendung eines Anti-CD81-Antikörpers für die Herstellung eines Arzneimittels zur Vorbeugung, Verbesserung oder Behandlung einer chronisch-entzündlichen Darmerkrankung (CED).

2. Verwendung gemäß Anspruch 1, wobei das CD81 ein Säuger-CD81 ist.

3. Verwendung gemäß Anspruch 1 oder 2, wobei der Anti-CD81-Antikörper ein monoclonaler Antikörper ist.

4. Verwendung gemäß einem der Ansprüche 1 bis 3, wobei die chronisch-entzündliche Darmerkrankung Colitis ulcerosa ist.

5. Verwendung gemäß einem der Ansprüche 1 bis 3, wobei die chronisch-entzündliche Darmerkrankung Morbus Crohn ist.

6. Anti-CD81-Antikörper zur Verwendung in der Vorbeugung, Verbesserung oder Behandlung einer chronisch-entzündlichen Darmerkrankung (CED).

7. Anti-CD81-Antikörper zur Verwendung nach Anspruch 6, wobei das CD81 ein Säuger-CD81 ist.

8. Anti-CD81-Antikörper zur Verwendung nach Anspruch 6 oder 7, wobei der Antikörper ein monoclonaler Antikörper ist.

9. Anti-CD81-Antikörper zur Verwendung nach einem der Ansprüche 6 bis 8, wobei die chronisch-entzündliche Darmerkrankung Colitis ulcerosa ist.

10. Anti-CD81-Antikörper zur Verwendung nach einem der Ansprüche 6 bis 8, wobei die chronisch-entzündliche Darmerkrankung Morbus Crohn ist.

**Revendications**

1. Utilisation d'un anticorps anti-CD81 pour la préparation d'une composition pharmaceutique destinée à prévenir, améliorer ou traiter une maladie inflammatoire intestinale (IBD).

2. Utilisation selon la revendication 1, dans laquelle le CD81 est un CD81 de mammifère.

3. Utilisation selon la revendication 1 ou 2, dans laquelle l'anticorps anti-CD81 est un anticorps monoclonal.

4. Utilisation selon l'une quelconque des revendications 1 à 3, dans laquelle ladite maladie inflammatoire intestinale est la rectocolite ulcéro-hémorragique.

5. Utilisation selon l'une quelconque des revendications 1 à 3, dans laquelle ladite maladie inflammatoire intestinale est la maladie de Crohn.

6. Anticorps anti-CD81 destiné à être utilisé pour prévenir, améliorer ou traiter une maladie inflammatoire intestinale (IBD).

7. Anticorps anti-CD81 destiné à une utilisation selon la revendication 6, où le CD81 est un CD81 de mammifère.

8. Anticorps anti-CD81 destiné à une utilisation selon la revendication 6 ou 7, où ledit anticorps est un anticorps monoclonal.

9. Anticorps anti-CD81 destiné à une utilisation selon l'une quelconque des revendications 6 à 8, où ladite maladie inflammatoire intestinale est la rectocolite ulcéro-hémorragique.

10. Anticorps anti-CD81 destiné à une utilisation selon l'une quelconque des revendications 6 à 8, où ladite maladie inflammatoire intestinale est la maladie de Crohn.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002161084 A
- JP 1059878 A
- WO 9312227 A
- WO 9203918 A
- WO 9402602 A
- WO 9425585 A
- WO 9634096 A
- WO 9633735 A
- EP 125023 A
- WO 9219759 A
- EP 239400 A
- WO 9630394 A
- WO 9411523 A
- JP 2003304264 A


### Non-patent literature cited in the description


- *Lancet.,* 1993, vol. 342, 173-174
- *Current Opinion in Anti-inflammatory & Immunomodulatory Investigational Drugs,* 2000, vol. 2, 272-274
- *Science,* 1998, vol. 282, 938-941
- *Clinical & Experimental Immunology,* 2000, vol. 128 (2), 353-8
- *Gastroenterologie Clinique et Biologique,* 2000, vol. 24 (1), 77-81
- IUPAC-IUB Communication on Biological Nomenclature. *Eur. J. Biochem.,* 1984, vol. 138, 9
- *Proc. Natl. Acad. Sci. USA,* 1993, vol. 90, 5873-5877
- **T. Maniatis et al.** Molecular Cloning. CSH Laboratory, 1983
- **DM. Glover.** DNA Cloning. IRL PRESS, 1985
- **Behr S et al.** *J. Exp Med,* 1995, vol. 182, 1191-1199
- Molecular Cloning. Cold Spring Harbor Laboratory Press, 1989
- *Gene,* 1991, vol. 108, 193-199
- *J. Virol.,* 1973, vol. 52, 456-467
- *Cell,* 1990, vol. 63, 185-194
- *J Exp Med,* 1995, vol. 182, 1191-1199
- In vivo models of Inflammation. **D. W. Morgans.** In vivo models of Inflammation. Birkhauser Verlag, 1999
- *Gastroenterology,* 1994, vol. 107, 1726-1735
- *Gastroenterology,* 1989, vol. 96, 795-803
- **Kearney, J.F. et al.** *J. Immunol.,* 1979, vol. 123, 1548-1550
- *Current Topics in Microbiology and Immunology,* 1978, vol. 81, 1-7
- **Kohler, G. ; Milstein, C.** *Eur. J. Immunol.,* 1976, vol. 6, 511-519
- **Margulies, D. H. et al.** *Cell,* 1976, vol. 8, 405-415
- **Shulman, M. et al.** *Nature,* 1978, vol. 276, 269-270
- **de St. Groth, S. F. et al.** *J. Immunol. Methods,* 1980, vol. 35, 1-21
- **Trowbridge, I.S.** *J. Exp. Med.,* 1978, vol. 148, 313-323
- **Galfre, G. et al.** *Nature,* 1979, vol. 277, 131-133
- **Kohler, G. ; Milstein, C.** *Methods Enzymol.,* 1981, vol. 73, 3-46
- **Borrebaeck C.A.K. ; Larrick J.4V.** THERAPEUTIC MONOCLONAL ANTIBODIES. MACMILLAN PUBLISHERS LTD, 1990
- **Chirgwin, J.M. et al.** *Biochemistry,* 1979, vol. 18, 5294-5299
- **Chomczynski, P. et al.** *Anal. Biochem.,* 1987, vol. 162, 156-159
- **Frohman, M.A. et al.** *Proc. Natl. Acad. Sci. U.S.A.,* 1988, vol. 85, 8998-9002
- **Belyavsky, A. et al.** *Nucleic Acids Res.,* 1989, vol. 17, 2919-2932
- **Sato, K. et al.** *Cancer Res.,* 1993, vol. 53, 851-856
- **Mulligan et al.** *Nature,* 1979, vol. 277, 108
- **Mizushima et al.** *Nucleic Acids Res.,* 1990, vol. 18, 5322
- **Ward et al.** *Nature,* 1989, vol. 341, 544-546
- *FASEB J.,* 1992, vol. 6, 2422-2427
- **Better et al.** *Science,* 1988, vol. 240, 1041-1043
- **Lei, S. P. et al.** *J. Bacteriol.,* 1987, vol. 169, 4379
- **Vicki Glaser.** *SPECTRUM Biotechnology Applications,* 1993
- **Ebert, K.M. et al.** *Bio/Technology,* 1994, vol. 12, 699-702

- **Maeda, S. et al.** *Nature,* 1985, vol. 315, 592-594
- **Julian, K. -C. Ma et al.** *Eur. J. Immunol.,* 1994, vol. 24, 131-138
- **Co, M. S. et al.** *J. Immunol.,* 1994, vol. 152, 2968-2976
- **Better, M. ; Horwitz, A.H.** Methods in Enzymology. Academic Press, Inc, 1989, vol. 178, 476-496
- **Plueckthun, A. ; Skerra, A.** *Methods in Enzymology,* 1989, vol. 178, 476-496
- **Lamoyi, E.** *Methods in Enzymology,* 1986, vol. 121, 652-663
- **Rousseaux, J. et al.** *Methods in Enzymology,* 1986, vol. 121, 663-669
- **Bird, R. E. et al.** *TIBTECH,* 1991, vol. 9, 132-137
- **Huston, J.S. et al.** *Proc. Natl. Acad. Sci. U. S.A.,* 1988, vol. 85, 5879-5883
- *Pharm Tech Japan,* 1992, vol. 8, 247, 395
- *Nikkei Science,* April 1994, 20-45
- *Gekkan Yakuji,* 1994, vol. 36 (1), 23-48
- *Jikken Igaku Zokan,* 1994, vol. 12 (15
- *Nature,* 2001, vol. 411, 494
- *TRENDS in Molecular Medicine,* 2001, vol. 7, 221
- **Prasad SS et al.** *Brain Res.,* 1994, vol. 639, 73-84
- **Berger ; Kimmel.** Guide to Molecular Cloning Techniques Methods in Enzymology. Academic Press, 1987, vol. 152
- **BRENDAN J. CLASSON et al.** *J Exp Med,* 1989, vol. 169, 1497-1502
- Current Protocol in Molecular Biology. 2000
- Current protocols in Molecular Biology. John Wiley and Sons, 1987
- *J. Exp. Med,* 1995, vol. 182, 1281-1290
- Nihon Shokaki Kanren Gakkai Shukan. Shokaki Byo Poster. Shiga Medical Collage, 2003, vol. 337